# FASCICULE DE BREVET EUROPEEN

(11) **EP 0 605 040 B1**
(45) Date de publication et mention de la délivrance du brevet: **11.08.1999**
(21) Numéro de dépôt: 93203593.4
(22) Date de dépôt: 20.12.1993
(51) Int. Cl.: C12N 15/56, C12N 15/75, C12N 9/44, C12P 19/16, C12N 1/21, C12N 1/20

(54) **Pullulanase, microorganismes la produisant, procédés de préparation de cette pullulanase et utilisations de celle-ci**
Pullulanase, Mikroorganismen die sie produzieren, Verfahren zur Herstellung und ihre Anwendung
Pullulanase, micro-organisms producing the same, method for preparation thereof as well as its use

(30) Priorité: 28.12.1992 BE 9201156; 15.07.1993 BE 9300744; 19.11.1993 BE 9301278
(43) Date de publication de la demande: 06.07.1994
(73) Titulaire: GENENCOR INTERNATIONAL, INC., Rochester, New York 14618 (US)
(72) Inventeur: DeWeer, Philippe, B-9300 Aalst (BE); Amory, Antoine, B-1330 Rixensart (BE)
(74) Mandataire: Kiddle, Simon John

(56) Documents cités:
- EP-A- 0 405 283
- JOURNAL OF BACTERIOLOGY vol. 170, no. 4 , Avril 1988 pages 1554 - 1559 TAKASHI KURIKI ET AL. 'New type of pullulanase from Bacillus stearothermophilus and molecular cloning and expression of hte gene in Bacillus subtilis'

## Description

L'invention concerne une nouvelle pullulanase. L'invention concerne également une nouvelle souche de microorganismes produisant cette pullulanase et les procédés de préparation de cette pullulanase. L'invention concerne également les utilisations et les compositions comprenant celle-ci. L'invention concerne également une molécule d'ADN contenant le gène de cette pullulanase et un vecteur d'expression contenant cette molécule d'ADN, utile pour exprimer la pullulanase dans les souches de Bacillus.

L'amidon, dont les constituants essentiels sont l'amylose et l'amylopectine, peut être converti en sucres simples par un procédé enzymatique réalisé en deux étapes : une étape de liquéfaction de l'amidon et une étape de saccharification de l'amidon liquéfié. En vue d'atteindre un taux de conversion élevé de l'amidon, il a déjà été proposé d'ajouter lors de la saccharification de l'amidon liquéfié une enzyme hydrolysant les liaisons glucosidiques α-1,6, comme, par exemple, une pullulanase.

Dans le brevet européen 0 063 909, on décrit une enzyme dite débranchante, c'est-à-dire capable d'hydrolyser les liaisons glucosidiques α-1,6 dans l'amylopectine, qui présente une activité pullulanase et possède un optimum d'activité à un pH de 4-5 à 60 °C. Cette enzyme dérive d'une souche de Bacillus acidopullulyticus.

Une pullulanase similaire, qui dérive d'une souche de Bacillus, est également décrite (J. Jpn. Soc. Starch Sci., 1983, vol. 30, n° 2, pages 200-211). Cette pullulanase présente un optimum d'activité à un pH de 5,0 et à une température de 60 °C. Cependant, elle est inactivée à un pH inférieur à 4,2 à une température de 60 °C.

Par ailleurs, dans le brevet Etats-Unis 5,055,403, on a proposé une pullulanase présentant une activité enzymatique en milieu acide et dérivée d'une souche de Bacillus naganoensis. Cette enzyme présente un maximum d'activité à un pH d'environ 5 mesurée à 60 °C et un maximum d'activité à une température d'environ 62,5 °C mesurée à un pH de 4,5.

De plus, le clonage d'une néopullulanase de Bacillus stearothermophilus dans Bacillus subtilis est également connu (Journal of bacteriology, 1988, vol. 170, n° 4, pages 1554-1559). Cependant, cette néopullulanase n'hydrolyse que partiellement l'amidon.

Quoiqu'actives à pH acide et à une température d'environ 60 °C et dès lors utilisables lors de la saccharification de l'amidon liquéfié, les pullulanases de l'art antérieur présentent l'inconvénient d'être très peu stables dans de telles conditions de température et de pH, leur durée de demi-vie à une température de 60 °C et à un pH d'environ 4,5 en l'absence de substrat ne dépassant pas quelques dizaines de minutes.

En conséquence il y a actuellement un besoin pour une pullulanase utilisable lors de la saccharification de l'amidon liquéfié, très stable dans une large gamme de température et de pH, et en particulier à une température d'environ 60 °C et à un pH d'environ 4,5.

La présente invention a pour but de fournir une nouvelle pullulanase, active à pH acide, présentant une thermostabilité à pH acide très largement supérieure à celle des pullulanases de l'art antérieur et une durée de demi-vie de plusieurs heures sous les conditions précitées.

La présente invention a également pour but d'identifier, isoler et fournir une souche, et en particulier une souche de Bacillus, qui produit naturellement ladite pullulanase.

La présente invention a également pour but d'isoler et de fournir une séquence de nucléotides codant pour ladite pullulanase.

La présente invention a également pour but de préparer et fournir un vecteur d'expression et un vecteur d'intégration chromosomique contenant la séquence de nucléotides codant pour ladite pullulanase.

La présente invention a également pour but de préparer et fournir un hôte de Bacillus transformé avec le vecteur d'expression ou le vecteur d'intégration contenant la séquence de nucléotides de la souche de Bacillus codant pour ladite pullulanase.

A cet effet l'invention concerne une pullulanase produite par un Bacillus, et plus particulièrement par un microorganisme aérobie et non thermophile tel que le Bacillus deramificans. On utilise de préférence le Bacillus deramificans T 89.117D ou un dérivé ou mutant de cette souche.

De préférence la pullulanase, isolée et purifiée, est constituée d'un seul type de polypeptide, ayant un poids moléculaire d'environ 100 (± 10) kDa.

De plus, la séquence N-terminale (SEQ ID NO:1) de ladite pullulanase est la suivante dans le sens amino-carboxy et de gauche à droite :

Asp Gly Asn Thr Thr Thr Ile Ile Val His
1               5                   10
Tyr Phe Cys Pro Ala Gly Asp Tyr Gln Pro
                15                  20

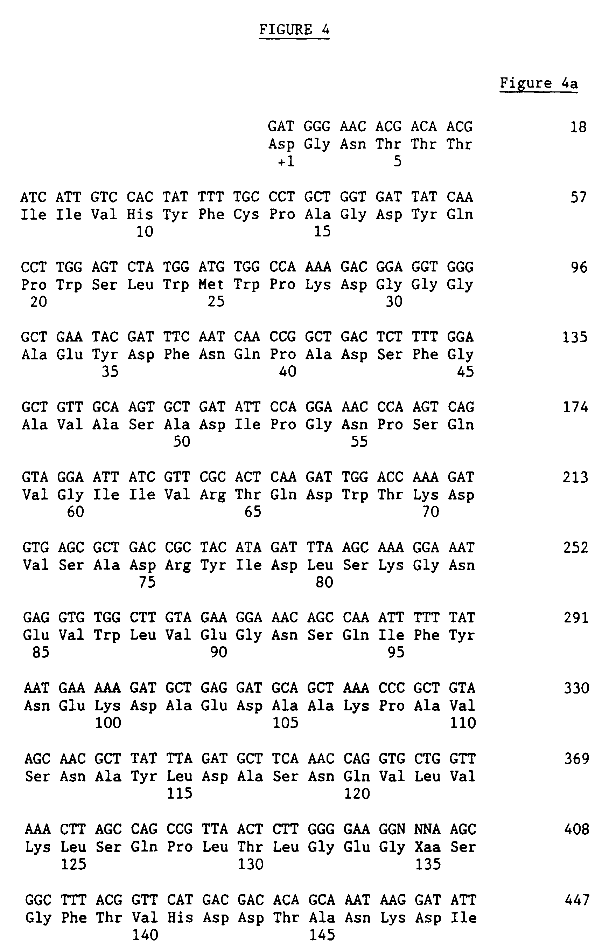

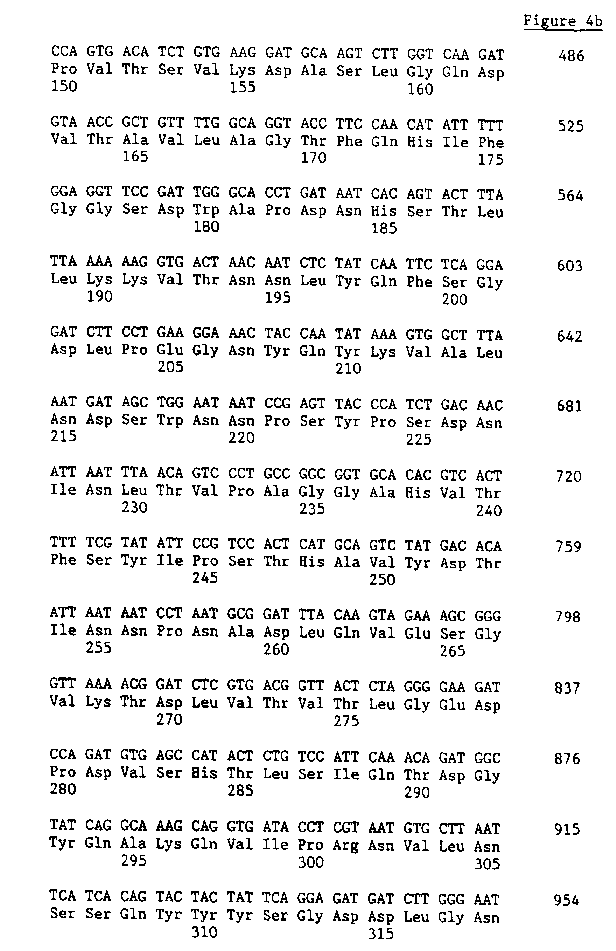

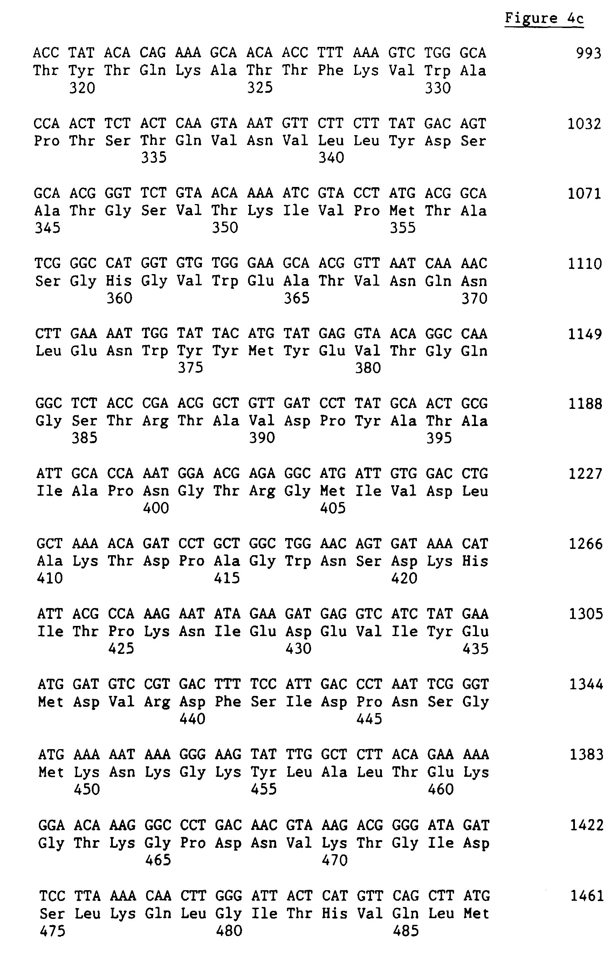

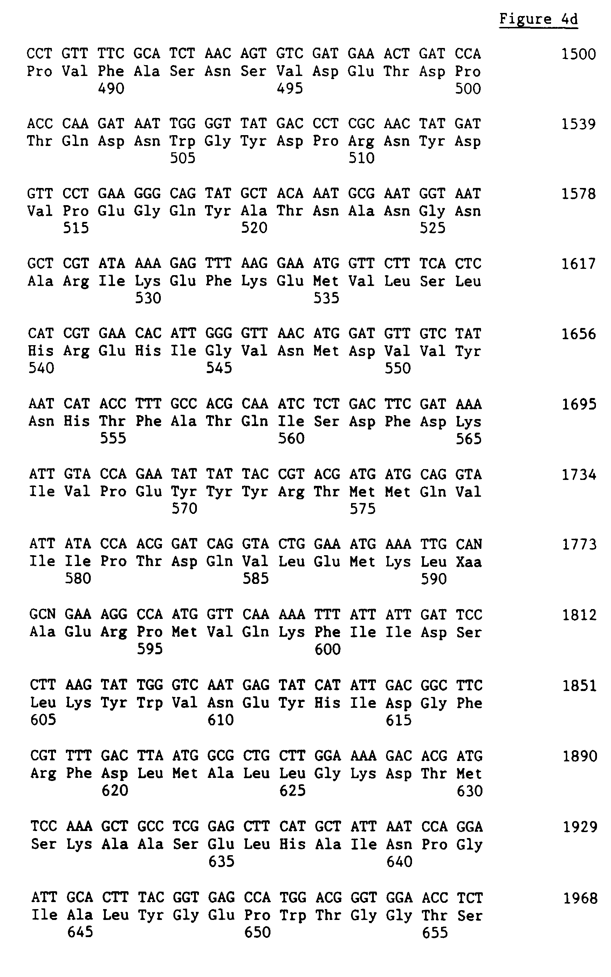

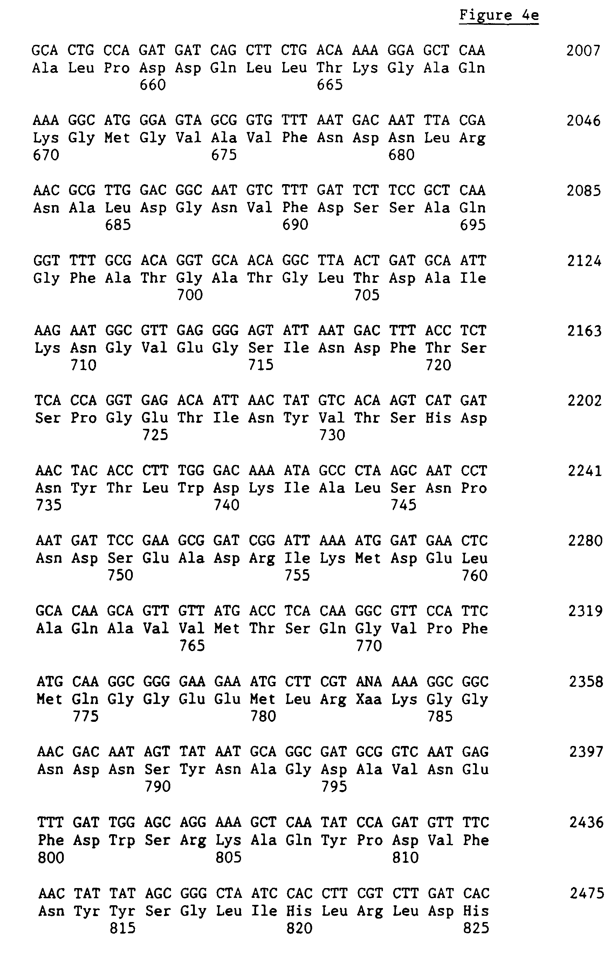

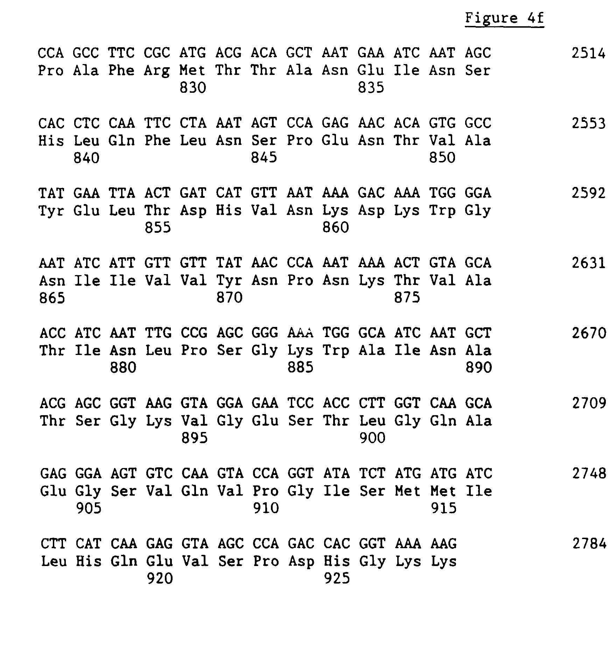

L'invention concerne une pullulanase, isolée et purifiée, comprenant la séquence d'acides aminés de 1 à 928 acides aminés (SEQ ID NO:11) ou une séquence modifiée dérivée de celle-ci. Cette séquence est la séquence complète en acides aminés de ladite pullulanase, telle qu'illustrée à la figure 4 (4a à 4f).

La séquence complète de nucléotides (SEQ ID NO:10) codant pour la pullulanase, ainsi que sa traduction en acides aminés, est donnée à la figure 4.

De manière particulièrement préférée, ladite pullulanase a un point isoélectrique compris entre 4,1 et 4,5.

La pullulanase selon l'invention est thermostable et active dans une large gamme de température. La pullulanase est active à pH acide.

Ladite pullulanase est capable de catalyser l'hydrolyse des liaisons glucosidiques α-1,6 présentes aussi bien dans l'amylopectine que dans le pullulane. C'est donc une enzyme dite déramifiante ou débranchante. De préférence ladite pullulanase est capable d'hydrolyser les liaisons glucosidiques de type α-1,6 dans l'amylopectine.

De préférence, la pullulanase selon l'invention dégrade le pullulane en maltotriose et l'amylopectine en amylose.

De plus, la pullulanase de la présente invention hydrolyse l'amylopectine en formant des oligosaccharides (maltooligosaccharides). Lors de cette hydrolyse, on observe la formation d'oligosaccharides constitués d'environ 13 unités de glucose (degré de polymérisation de 13, cette molécule est aussi appelée "chaîne A"), suivie de la formation d'oligosaccharides constitués d'environ 47 unités de glucose (degré de polymérisation de 47, cette molécule est aussi appelée "chaîne B").

La définition des oligosaccharides avec des chaînes A et B est faite par référence à D.J. MANNERS ("Structural Analysis of Starch components by Debranching Enzymes" dans "New Approaches to research on Cereal Carbohydrates", Amsterdam ,1985, p. 45-54) et B.E. ENEVOLDSEN ("Aspects of the fine structure of starch" dans "New Approaches to research on Cereal Carbohydrates", Amsterdam, 1985, p. 55-60).

De préférence, la pullulanase de la présente invention hydrolyse l'amylopectine de pomme de terre. Cette hydrolyse peut être réalisée avec une suspension aqueuse d'amylopectine en présence de la pullulanase dans les conditions optimales d'activité de la pullulanase, c'est-à-dire à une température d'environ 60 °C et à un pH d'environ 4,3.

La pullulanase de la présente invention catalyse la réaction de condensation du maltose en formant des tétraholosides (oligosaccharides ayant 4 unités glucose).

La pullulanase de l'invention a une durée de demi-vie d'environ 55 heures mesurée à une température d'environ 60 °C dans une solution tamponnée à un pH d'environ 4,5 et en l'absence de substrat.

Par durée de demi-vie, on entend que la pullulanase montre une activité enzymatique relative d'au moins 50 % mesurée après une incubation de 55 heures à une température d'environ 60 °C dans une solution tamponnée à un pH d'environ 4,5 et en l'absence de substrat.

La pullulanase selon l'invention est thermostable à pH acide. En effet, la pullulanase selon l'invention montre une activité enzymatique relative d'au moins 55 % mesurée après une incubation de 40 heures à une température de 60 °C dans une solution tamponnée à un pH d'environ 4,5 et en l'absence de substrat. Elle montre une activité enzymatique relative d'au moins 70 % mesurée après une incubation de 24 heures sous ces mêmes conditions.

Par activité enzymatique relative, on entend le rapport entre l'activité enzymatique, mesurée au cours d'un essai réalisé dans des conditions données de pH, température, substrat et durée, et l'activité enzymatique maximale mesurée au cours de ce même essai, l'activité enzymatique étant mesurée à partir de l'hydrolyse du pullulane et l'activité enzymatique maximale étant fixée arbitrairement à la valeur de 100.

La pullulanase selon l'invention est par ailleurs stable dans une large gamme de pH acides.

Sous les conditions décrites ci-après, elle est active à un pH supérieur ou égal à 3. En effet, ladite pullulanase montre une activité enzymatique relative d'au moins 85 % mesurée après une incubation de 60 minutes à une température d'environ 60 °C en l'absence de substrat et dans une gamme de pH supérieur ou égal à environ 3,5.

Sous les conditions décrites ci-après, elle est active à un pH inférieur ou égal à 7. En effet, ladite pullulanase montre une activité enzymatique relative d'au moins 85 % mesurée après une incubation de 60 minutes à une température d'environ 60 °C en l'absence de substrat et dans une gamme de pH inférieur ou égal à environ 5,8.

De préférence, elle montre une activité enzymatique relative supérieure à 90 % mesurée dans une gamme de pH compris entre environ 3,8 et environ 5 sous ces mêmes conditions.

La pullulanase selon l'invention développe une activité enzymatique optimale mesurée à une température d'environ 60 °C dans une gamme de pH supérieur à 4,0. La pullulanase selon l'invention développe une activité enzymatique optimale mesurée à une température d'environ 60 °C dans une gamme de pH inférieur à 4,8. De préférence ladite pullulanase développe une activité enzymatique optimale mesurée à une température d'environ 60 °C à un pH d'environ 4,3.

La pullulanase selon l'invention développe par ailleurs une activité enzymatique optimale, mesurée à un pH d'environ 4,3, dans une gamme de température comprise entre 55 et 65 °C, et plus particulièrement à 60 °C.

La pullulanase selon l'invention développe une activité enzymatique de plus de 80 % de l'activité enzymatique maximale (l'activité enzymatique maximale étant mesurée à une température de 60 °C et à un pH de 4,3) dans une gamme de pH compris entre environ 3,8 et environ 4,9 pour une température d'environ 60 °C.

La pullulanase selon l'invention possède par ailleurs toutes les propriétés adéquates compatibles avec les conditions industrielles réelles de saccharification de l'amidon. Ces propriétés sont un optimum de pH inférieur à 5, un optimum de température aux alentours de 60 °C et une bonne stabilité de l'enzyme dans ces conditions de pH acide et de température élevée. Le milieu acide est imposé par l'utilisation simultanée de la glucoamylase et de la pullulanase lors de la saccharification industrielle de l'amidon. En effet la glucoamylase utilisée pour la saccharification de l'amidon est généralement produite par un champignon et notamment par une souche d'Aspergillus, tel que Aspergillus niger, Aspergillus awamori ou Aspergillus foetidus. Les conditions idéales adaptées à la saccharification d'amidon liquéfié en présence d'une glucoamylase sont une température d'environ 60 °C et un pH d'environ 4,0 à 4,5. Ceci est notamment le cas pour la glucoamylase vendue sous les marques DIAZYME® L-200 par SOLVAY ENZYMES (Elkhart, Etats-Unis) et OPTIDEX® par SOLVAY ENZYMES (Hanovre, Allemagne). Par ailleurs l'étape de saccharification dure plusieurs heures, en général de 40 à 60 heures, il est essentiel que les enzymes utilisées soient stables, actives et efficaces tout au long de cette étape, ces enzymes doivent donc présenter une thermostabilité élevée en milieu acide et une durée de demi-vie la plus longue possible. C'est pourquoi la pullulanase de la présente invention est plus efficace que les pullulanases connues.

La présente invention concerne également un procédé pour la production d'une pullulanase comprenant la culture d'une bactérie aérobie (et non thermophile) capable de produire la pullulanase dans un milieu nutritif approprié contenant des sources de carbone et d'azote et des sels minéraux sous condition d'aérobiose et la récolte de la pullulanase ainsi obtenue. Ce milieu de culture peut être solide ou liquide. De préférence le milieu de culture est liquide.

La présente invention concerne également un procédé pour la production d'une pullulanase comprenant la culture de la souche de Bacillus deramificans T 89.117D (LMG P-13056) ou un dérivé de cette souche capable de produire la pullulanase dans un milieu nutritif approprié contenant des sources de carbone et d'azote et des sels minéraux sous condition d'aérobiose et la récolte de la pullulanase ainsi obtenue.

Les conditions de culture de ces bactéries telles que composants du milieu de culture, paramètres de culture, température, pH, aération, agitation, sont bien connues de l'homme du métier.

Les sources de carbone du milieu de culture sont habituellement choisies parmi l'amidon, l'amidon partiellement hydrolysé, l'amidon soluble, des oligosaccharides, le glucose, l'amylose, l'amylopectine ou un mélange de deux ou plusieurs de ceux-ci. Les sources de carbone du milieu de culture sont de préférence choisies parmi l'amidon partiellement hydrolysé, le pullulane, le glucose ou un mélange de ceux-ci. De bons résultats ont été obtenus avec le glucose et l'amidon partiellement hydrolysé. Les sources d'azote du milieu de culture sont habituellement choisies parmi l'extrait de levure, la farine de soja, la farine de graines de coton, la farine de poisson, la gélatine, la farine de pomme de terre ou un mélange de deux ou plusieurs de ceux-ci. Les sources d'azote du milieu de culture sont de préférence choisies parmi l'extrait de levure, la farine de soja ou un mélange de ceux-ci. De bons résultats ont été obtenus avec l'extrait de levure. Les sels minéraux du milieu de culture sont généralement choisis pour les anions parmi chlorure, carbonate, phosphate, sulfate et pour les cations parmi potassium, sodium, ammonium, magnésium, calcium ou un mélange de deux ou plusieurs de ceux-ci. De bons résultats ont été obtenus avec un mélange des sels suivants KH₂PO₄, K₂HPO₄.3H₂O, (NH₄)₂SO₄, MgCl₂.6H₂O et CaCl₂.2H₂O.

La culture est généralement conduite à une température comprise entre 20 et 45 °C et de préférence entre 25 et 40 °C.

La culture est généralement conduite à un pH compris entre 3,5 et 6 et de préférence entre 4 et 6.

La culture est conduite sous condition d'aérobiose en présence d'air ou d'oxygène et sous agitation.

Les techniques de récolte de la pullulanase produite sont bien connues de l'homme du métier. Habituellement, on emploie la centrifugation, l'ultrafiltration, l'évaporation, la précipitation, la filtration, la microfiltration, la cristallisation ou une combinaison de l'une ou l'autre de ces techniques telle qu'une centrifugation suivie d'une ultrafiltration.

La pullulanase peut ensuite être purifiée, si nécessaire. Les techniques de purification d'enzymes sont connues de l'homme du métier, telles que notamment la précipitation à l'aide d'un sel, tel que le sulfate d'ammonium, ou de solvant, tel que principalement l'acétone.

La pullulanase peut également être séchée par atomisation ou lyophilisation.

La présente invention concerne également l'identification et la fourniture d'une nouvelle bactérie isolée aérobie produisant la pullulanase. Généralement elle appartient à la famille des Bacillaceae. De préférence elle appartient au genre Bacillus. De manière particulièrement préférée, ledit Bacillus est la souche de Bacillus deramificans T 89.117D ou un dérivé ou mutant de cette souche.

Par dérivé ou mutant de cette souche, on entend toute bactérie modifiée naturellement ou artificiellement. Les dérivés de cette souche peuvent être obtenus par les techniques connues de modification telles que rayonnement ultra-violet, rayons X, agents mutagènes ou génie génétique.

La souche de Bacillus deramificans T 89.117D a été déposée à la collection nommée BELGIAN COORDINATED COLLECTIONS OF MICROORGANISMS (LMG culture collection, Université de Gand, Laboratoire de Microbiologie - K.L. Ledeganckstraat 35, B-9000 Gand, Belgique) conformément au Traité de Budapest sous le numéro LMG P-13056 le 9 décembre 1992. L'invention concerne donc une culture isolée et purifiée de Bacillus deramificans T 89.117D et une culture dérivée ou mutée de celle-ci.

La souche de la présente invention a été identifiée par ses caractéristiques biochimiques : bactérie Gram positif, aérobie qui se présente sous le forme de bâtonnet, elle forme une endospore.

L'invention concerne également l'isolement et la fourniture d'une molécule d'ADN comprenant une séquence de nucléotides (SEQ ID NO:10) codant pour la pullulanase de Bacillus deramificans T 89.117D (LMG P-13056) ou une séquence modifiée dérivée de celle-ci. De préférence cette molécule d'ADN comprend tout le gène de la pullulanase de Bacillus deramificans T 89.117D. Par tout le gène de la pullulanase, on entend au moins le (ou les) promoteur(s) de transcription, la (ou les) séquence(s) signal, la séquence de nucléotides codant pour la pullulanase mature et le (ou les) terminateur(s) de transcription.

La molécule d'ADN, selon l'invention, comprend au moins la séquence de nucléotides (SEQ ID NO:10) codant pour la pullulanase mature de Bacillus deramificans T 89.117D (LMG P-13056) et sa séquence signal (préséquence) (SEQ ID NO:13). De préférence cette molécule d'ADN comprend tout le gène de la pullulanase de Bacillus deramificans T 89.117D. De bons résultats ont été obtenus avec une molécule d'ADN comprenant la séquence de nucléotides (SEQ ID NO:8). La séquence de nucléotides (SEQ ID NO:8) est constituée de, dans le sens amino-carboxy et de gauche à droite, la séquence de nucléotides (SEQ ID NO:14), la séquence de nucléotides (SEQ ID NO:13), la séquence de nucléotides (SEQ ID NO:10) et la séquence de nucléotides (SEQ ID N0:15).

La pullulanase de l'invention est synthétisée sous forme d'un précurseur contenant une séquence additionnelle de 29 acides aminés (SEQ ID N0:12).

L'invention concerne également une pullulanase modifiée, c'est-à-dire une enzyme dont la séquence en acides aminés diffère de celle de l'enzyme sauvage par au moins un acide aminé. Ces modifications peuvent être obtenues par des techniques classiques de mutagénèse sur l'ADN, telles que l'exposition à des rayonnements ultra-violets, à des produits chimiques tels que le nitrite de sodium ou l'o-methylhydroxylamine ou par des techniques de génie génétique comme par exemple la mutagénèse dirigée ou la mutagénèse aléatoire.

L'invention concerne également une pullulanase mutée obtenue par modification de la séquence de nucléotides du gène qui code pour la pullulanase définie ci-dessus. Les techniques d'obtention de telles pullulanases mutées sont connues de l'homme du métier et sont notamment décrites dans Molecular Cloning - a laboratory manual - SAMBROOK, FRITSCH, MANIATIS - second edition, 1989, au chapitre 15.

L'invention concerne également la préparation et la fourniture d'un vecteur d'expression contenant la molécule d'ADN qui comprend la séquence de nucléotides qui code pour la pullulanase de Bacillus deramificans T 89.117D. De préférence la molécule d'ADN comprend le gène de structure qui code pour la pullulanase mature de Bacillus deramificans T 89.117D. De manière particulièrement préférée ce vecteur est le vecteur pUBDEBRA1. De bons résultats ont également été obtenus avec le vecteur pUBCDEBRA11.

Par vecteur d'expression on entend toute séquence d'ADN qui comprend un réplicon et d'autres régions d'ADN (séquences de nucléotides) et qui est fonctionnelle indépendamment de l'hôte comme une unité d'expression génique complète.

Par unité d'expression génique complète, on entend le gène de structure et la ou les région(s) du promoteur et la ou les région(s) de régulation nécessaire pour la transcription et la traduction. Par gène de structure on entend la séquence codante qui est utilisée pour la transcription en ARN et permet la synthèse de la protéine par l'hôte.

Le vecteur d'expression préféré est le vecteur pUBDEBRA1. Ce vecteur contient le gène qui code pour la pullulanase de la souche de Bacillus deramificans T 89.117D selon l'invention. Ce vecteur peut être introduit dans un hôte approprié. Généralement cet hôte est une souche de Bacillus. De préférence cet hôte est une souche de Bacillus licheniformis. De manière particulièrement préférée cet hôte est une souche de Bacillus licheniformis SE2. D'excellents résultats ont été obtenus avec ce vecteur lorsqu'il est introduit dans la souche de Bacillus licheniformis SE2 delap1, utilisée comme hôte.

L'invention concerne également la préparation et la fourniture d'un vecteur d'intégration chromosomique contenant la molécule d'ADN qui comprend la séquence de nucléotides qui code pour la pullulanase de Bacillus deramificans T 89.117D. De préférence la molécule d'ADN comprend le gène de structure qui code pour la pullulanase mature de Bacillus deramificans T 89.117D. De manière particulièrement préférée, ce vecteur d'intégration chromosomique est le vecteur pUBCDEBRA11DNSI.

La présente invention concerne également des souches recombinantes dans lesquelles ledit gène codant pour la pullulanase est introduit par les techniques de génie génétique. Le gène peut être introduit sur un plasmide par un vecteur d'expression ou intégré dans le chromosome de l'hôte en une ou plusieurs copies par un vecteur d'intégration chromosomique.

L'invention concerne également les souches de microorganismes différentes de l'organisme producteur de départ, dans lesquelles les nucléotides codant pour la pullulanase sont introduits par transformation, soit sous forme intégrée dans l'ADN chromosomique, soit sous forme autoréplicative (plasmide).

L'invention concerne la souche transformée de Bacillus licheniformis comprenant la molécule d'ADN décrite ci-dessus. L'invention concerne la souche transformée de Bacillus licheniformis comprenant le vecteur d'expression ou le vecteur d'intégration chromosomique qui comprend cette molécule d'ADN. De préférence l'invention concerne la souche transformée de Bacillus licheniformis comprenant le vecteur d'expression pUBDEBRA1 ou le vecteur d'intégration chromosomique pUBCDEBRA11DNSI.

L'invention concerne également un procédé pour la préparation d'une pullulanase à partir d'un organisme recombinant, le procédé comprenant l'isolement d'un fragment d'ADN codant pour la pullulanase, l'insertion de ce fragment d'ADN dans un vecteur approprié, l'introduction de ce vecteur dans un hôte approprié ou l'introduction de ce fragment d'ADN dans le chromosome d'un hôte approprié, la culture de cet hôte, l'expression de la pullulanase et la récolte de la pullulanase. L'hôte approprié est choisi généralement parmi le groupe constitué des microorganismes Escherichia coli, Bacillus ou Aspergillus. Habituellement, l'hôte est choisi parmi les Bacillus. De préférence, l'hôte est choisi parmi les microorganismes du genre Bacillus (aérobies). De manière particulièrement préférée, l'hôte est choisi parmi les microorganismes Bacillus subtilis, Bacillus licheniformis, Bacillus alcalophilus, Bacillus pumilus, Bacillus lentus, Bacillus amyloliquefaciens ou Bacillus deramificans T 89.117D (LMG P-13056).

De bons résultats ont été obtenus lorsque l'hôte pour l'expression de la pullulanase selon la présente invention est une souche recombinante dérivée de Bacillus licheniformis, et de préférence la souche de Bacillus licheniformis SE2 delap1.

La souche de Bacillus licheniformis SE2 a été déposée le 21 juin 1993 à la collection nommée BELGIAN COORDINATED COLLECTIONS OF MICROORGANISMS (LMG culture collection, Gand, Belgique) conformément au Traité de Budapest sous le numéro LMG P-14034.

La souche transformée SE2 delap1 ainsi obtenue de Bacillus licheniformis SE2 diffère de la souche mère par le seul fait qu'elle ne contient pas dans son chromosome la séquence d'ADN qui code pour la protéase mature.

L'invention concerne également une pullulanase produite, de façon hétérologue, par un microorganisme du genre Bacillus qui contient un gène codant pour une protéase alcaline lorsqu'il est à l'état sauvage. De préférence ce microorganisme est une souche de Bacillus licheniformis comprenant la molécule d'ADN qui comprend la séquence de nucléotides qui code pour la pullulanase de Bacillus deramificans T 89.117D. De manière particulièrement préférée, le gène codant pour la protéase alcaline a été délété de cette souche de Bacillus. Cette souche est de préférence la souche de Bacillus licheniformis SE2 delap1.

Par produite de façon hétérologue, on entend une production qui n'est pas effectuée par le microorganisme naturel, c'est-à-dire le microorganisme qui contient à l'état sauvage le gène qui code pour la pullulanase.

La pullulanase selon l'invention a des débouchés multiples dans diverses industries, telles que, par exemples, les industries alimentaires, les industries pharmaceutiques ou les industries chimiques.

La pullulanase peut notamment être utilisée en boulangerie comme "anti-staling", c'est-à-dire comme additif pour éviter au pain de rassir au cours de sa conservation ou en brasserie au cours de la fabrication de bières à faible teneur en calories.

La pullulanase peut également être utilisée lors de la préparation d'aliments à faible teneur en calories dans lesquels l'amylose est utilisé comme substitut des matières grasses.

La pullulanase peut également être utilisée pour hydrolyser l'amylopectine et former des oligosacharides à partir de cette amylopectine.

La pullulanase peut également être utilisée pour former des tétraholosides à partir de maltose.

La pullulanase peut également être utilisée pour condenser des mono- ou oligo-saccharides en créant des liaisons de type alpha-1,6.

La pullulanase peut, par exemple, être utilisée pour clarifier les jus de fruit.

La pullulanase peut être utilisée pour la liquéfaction de l'amidon.

Pour les applications alimentaires, la pullulanase peut être immobilisée sur un support. Les techniques d'immobilisation d'enzymes sont bien connues de l'homme du métier.

La pullulanase selon l'invention convient particulièrement bien pour le traitement de l'amidon et du pullulane.

L'invention concerne l'utilisation de la pullulanase pour la saccharification de l'amidon liquéfié.

La présente invention concerne également l'utilisation de la pullulanase lors d'un procédé de dégradation d'amidon ou d'amidon partiellement hydrolysé comprenant une étape de saccharification de l'amidon ou de l'amidon partiellement hydrolysé en présence d'une pullulanase. Généralement ce procédé est effectué en présence d'une ou de plusieurs autres enzymes telles que glucoamylase, α-amylase, β-amylase, α-glucosidase ou d'autres enzymes saccharifiantes.

Etant donné ses propriétés biochimiques, la pullulanase de la présente invention permet d'effectuer l'étape de saccharification dans des conditions fortement acides, c'est-à-dire jusqu'à au moins un pH de 3,9. Ce pH est plus acide que celui acceptable par les pullulanases connues.

Etant donné ses propriétés biochimiques, la pullulanase de la présente invention permet d'effectuer l'étape de saccharification à des températures relativement élevées, c'est-à-dire jusqu'à au moins une température de 65 °C.

L'addition de la pullulanase de la présente invention dans le milieu de saccharification permet d'augmenter la teneur en glucose dans la composition finale obtenue et donc d'augmenter le rendement de la réaction.

De plus, l'addition de la pullulanase de la présente invention dans le milieu de saccharification permet de diminuer la durée de la saccharification.

La pullulanase de la présente invention permet d'atteindre un taux de conversion élevé de l'amidon.

Par ailleurs lors de l'étape de saccharification, il est possible de remplacer une grande partie (au moins 60 %) de la glucoamylase, normalement mise en oeuvre, par la pullulanase de la présente invention, et cela sans affecter le rendement en glucose. Ce remplacement est particulièrement avantageux, en effet il permet de réduire notablement la quantité de sous produits obtenus habituellement. La glucoamylase étant en faible proportion, elle est dans l'incapacité de catalyser la réaction de synthèse d'oligosaccharides (contenant des liaisons α-1,6) à partir de glucose; dans les conditions normales, la glucoamylase catalyse cette réaction inverse de synthèse d'oligosaccharides lorsque des concentrations élevées de dextrose sont atteintes dans le milieu de saccharification, ce qui limite le taux de conversion de l'amidon.

De plus, la pullulanase de la présente invention permet de mettre en oeuvre un milieu de saccharification concentré, c'est-à-dire un milieu ayant une teneur élevée en amidon liquefié. Ceci est avantageux d'un point de vue économique, en effet ceci permet de diminuer les frais d'évaporation.

La présente invention concerne également des compositions enzymatiques comprenant la pullulanase selon l'invention.

Les compositions comprenant la pullulanase de la présente invention peuvent être utilisées sous forme solide ou liquide.

La pullulanase est formulée selon les utilisations prévues. Des stabilisants ou des agents de conservation peuvent également être ajoutés aux compositions enzymatiques comprenant la pullulanase selon l'invention. Par exemple on peut stabiliser la pullulanase par l'addition de propylène glycol, d'éthylène glycol, de glycérol, d'amidon, de pullulane, d'un sucre tel que du glucose et du sorbitol, d'un sel tel que du chlorure de sodium, du chlorure de calcium, du sorbate de potassium et du benzoate de sodium ou d'un mélange de deux ou plusieurs de ces produits. De bons résultats ont été obtenus avec du propylène glycol. De bons résultats ont été obtenus avec un mélange d'amidon, de benzoate de sodium et de sorbate de potassium.

Les compositions enzymatiques, selon l'invention, peuvent également comprendre, en plus de la pullulanase, une ou plusieurs autres enzymes. De telles enzymes sont notamment des hydrolases d'hydrates de carbone, comme, par exemples, les glucoamylase, α-amylase, β-amylase, α-glucosidase, isoamylase, cyclomaltodextrin-glucotransférase, β-glucanase, glucose-isomérase, des enzymes saccharifiantes, des enzymes qui coupent les liens glucosidiques ou un mélange de deux ou plusieurs de celles-ci.

La présente invention concerne de préférence une composition enzymatique comprenant une glucoamylase et une pullulanase.

La figure 1 représente la carte de restriction du plasmide pUBDEBRA1.

La figure 2 représente la carte de restriction du plasmide pLD1.

La figure 3 représente la carte de restriction du plasmide pUBCDEBRA11DNSI.

La figure 4 (figures 4a à 4f) représente la séquence de nucléotides (SEQ ID NO:10) codant pour la pullulanase mature, ainsi que sa traduction en acides aminés (SEQ ID NO:11).

La figure 5 (figures 5a à 5g) représente la séquence nucléotidique (SEQ ID NO:8) du fragment d'ADN, depuis le site BamHI jusqu'au site PstI du plasmide pUBCDEBRA11, ainsi que la traduction en acides aminés (SEQ ID NO:9) des séquences signal et mature de la pullulanase. Les nucléotides qui n'ont pas été déterminés avec certitude, ont été représentés par le symbole N.

La signification des symboles et abréviations utilisés dans ces figures est rassemblée dans le tableau suivant.

| Symbole Abréviation | Signification |
|---|---|
| ORIEC | origine de réplication dans E. coli |
| REP | protéine nécessaire pour la réplication |
| ORI+ | origine de réplication du brin + |
| ORI- | origine de réplication du brin - |
| KMR | gène apportant la résistance à la kanamycine |
| BLMR | gène apportant la résistance à la bléomycine |
| AMPR | gène apportant la résistance à l'ampicilline |
| PP | pré-pro-séquence |
| BLIAPR | séquence codant pour la protéase alcaline de B. licheniformis |
| 5'BLIAPR | séquence 5' située en amont de la séquence codant pour la protéase alcaline de B. licheniformis |
| 3'BLIAPR | séquence 3' située en aval de la séquence codant pour la protéase alcaline de B. licheniformis |
| BDEPUL | séquence codant pour la pullulanase de B. deramificans |

La présente invention est illustrée par les exemples suivants.

### Exemple 1

### Isolement et caractérisation de la souche de Bacillus deramificans

La souche de Bacillus deramificans T89.117D a été isolée du sol sur un milieu nutritif gélosé et sélectionnée pour sa capacité de dégrader un dérivé coloré du pullulane connu sous le nom de l'AZCL-pullulane et vendu par la Société MEGAZYME.

Cette souche a été mise en culture à 37 °C dans le milieu de croissance MYE dont la composition est la suivante :
KH₂PO₄ 33 mM; K₂HPO₄.3H₂O 6 mM; (NH₄)₂SO₄ 45 mM; MgCl₂.6H₂O 1 mM; CaCl₂.2H₂O 1 mM; Extrait de levure 0,5 % (poids/volume); Glucose 0,5 % (poids/volume). Le pH du milieu est ajusté à pH 4,5 avec H₃PO₄.

Le milieu gélosé (MYE/agar) contient 2 % (poids/volume) agar en plus.

La souche de la présente invention a été identifiée par ses caractéristiques biochimiques : bactérie Gram positif, aérobie qui se présente sous le forme de bâtonnet, elle forme une endospore. Elle appartient donc au genre Bacillus.

Les cellules végétatives de cette souche en culture sur le milieu MYE à 37 °C ont une forme de bacille de taille 0,7 x 3,0-3,5 µm. La mobilité des cellules végétatives est faible.

Après une croissance de trois jours à 37 °C sur le milieu MYE, l'observation microscopique révèle la présence de sporanges (sub)terminales légèrement déformées et en forme d'ellipse.

Le test de la catalase est faiblement positif en présence de 10 % de peroxyde d'hydrogène. Le test de l'oxydase est positif en présence de 1 % de tétraméthyl-1,4-phénylène-diammoniumdichlorure.

Cette souche est aérobie, c'est-à-dire qu'elle se développe en aérobiose. Elle ne se développe pas en anaérobiose, c'est-à-dire sous une atmosphère de 84 % (v/v) N₂, 8 % (v/v) CO₂, 8 % (v/v) H₂ à 37 °C, par contre elle se développe en microanaérobiose, c'est-à-dire sous une atmosphère de 82.5 % (v/v) N₂, 6 % (v/v) O₂, 7.5 % (v/v) H₂, 4 % (v/v) CO₂ à 37 °C. L'abréviation % (v/v) représente un pourcentage exprimé en volume par volume.

Cette souche n'est pas thermophile. Elle présente un développement normal après incubation en milieu MYE à 20 °C, 30 °C, 37 °C et 45 °C, par contre elle ne se développe pas à 50 °C et 55 °C. Elle présente un développement normal après incubation en milieu MYE tamponné avec le tampon phosphate aux pH suivants pH 4,0, pH 4,5, pH 5,0 et pH 5,5, par contre elle ne se développe pas à pH 7,0. Elle présente un développement normal après incubation en milieu MYE en présence de NaCl aux concentrations de 2,0 % (p/v) et 3,5 % (p/v), présente un développement faible en présence de 5,0 % (p/v) de NaCl et ne se développe pas en présence de 7,0 % (p/v) de NaCl. L'abréviation % (p/v) représente un pourcentage exprimé en poids par volume.

Cette souche n'hydrolyse pas la caséine : en effet aucune zone de lyse n'a pu être observée après plus de 2 semaines d'incubation à 37 °C. Elle décompose la tyrosine faiblement, ne produit pas d'acétoine à partir de pyruvate et ne réduit pas le nitrate en nitrite ou en N₂.

La souche de Bacillus deramificans T89.117D selon l'invention est taxonomiquement différente de la souche de Bacillus acidopullulyticus décrite dans le brevet européen 0 063 909 et de la souche de Bacillus naganoensis décrite dans le brevet U.S. 5,055,403. La souche de Bacillus deramificans T89.117D présente une croissance à un pH compris entre 4,7 et 5,5, ne présente pas de croissance à un pH de 7,0, se développe en présence de 3,5 % (p/v) de NaCl, décompose la tyrosine et ne réduit pas le nitrate en nitrite.

La souche de Bacillus deramificans T89.117D a été déposée à la collection nommée BELGIAN COORDINATED COLLECTIONS OF MICROORGANISMS (LMG culture collection) sous le numéro LMG P-13056.

### Exemple 2

### Préparation de la pullulanase

La souche de Bacillus deramificans T89.117D est mise en culture dans un milieu (MYA) liquide dont la composition est identique à celle du milieu MYE à l'exception de la teneur en extrait de levure et du glucose remplacé par de l'amidon, c'est-à-dire :
Extrait de levure 2,5 % (p/v)
Amidon de pomme de terre 2,5 % (p/v).

La culture est réalisée sous agitation, avec une aération efficace, à une température de 37 °C.

Après 68 heures de culture, on sépare la pullulanase et la biomasse cellulaire par centrifugation (5000 tours par minute pendant 30 minutes, BECKMAN JA-10). La pullulanase produite par la souche de Bacillus deramificans T89.117D est extracellulaire.

Puis on concentre la pullulanase par ultrafiltration (Membrane AMICON S10 Y10), en vue d'obtenir une solution aqueuse concentrée de pullulanase.

On mesure l'activité enzymatique de la solution obtenue.

Une unité enzymatique de la pullulanase (PUN) est définie comme la quantité d'enzyme qui, à un pH de 4,5, à une température de 60 °C et en présence de pullulane, catalyse la libération de sucres réducteurs à raison de 1 µM d'équivalent glucose par minute.

La mesure de l'activité enzymatique pullulanase est réalisée selon le protocole suivant. 1 ml d'une solution de pullulane à 1 % dans un tampon acétate 50 mM à pH 4,5 est incubée à 60 °C pendant 10 minutes. 0,1 ml d'une solution de pullulanase correspondant à une activité comprise entre 0,2 et 1 PUN/ml y est ajoutée. La réaction est stoppée après 15 minutes par addition de 0,4 ml de NaOH 0,5 M. Le dosage des sucres réducteurs libérés se fait par la méthode de SOMOGYI-NELSON [J. Biol. Chem., 153 (1944) p. 375-380; J. Biol. Chem., 160 (1945),p. 61-68], et comme dans les autres exemples de cette demande.

Une deuxième méthode est utilisée pour effectuer les dosages de la pullulanase. La réaction enzymatique en présence de pullulane est effectuée selon les conditions de l'essai, puis est arrêtée par l'addition d'acide sulfurique (0,1 N). Les produits d'hydrolyse du pullulane sont ensuite soumis à une chromatographie HPLC (colonne HPX-87H de BIO-RAD; la phase mobile est constituée de H₂SO₄ 10 mM) afin de séparer les différents constituants. La quantité de maltotriose formé est estimée par mesure de la surface du pic obtenu.

L'activité dite débranchante, c'est-à-dire l'hydrolyse des liaisons glucosidiques α-1,6 présentes dans l'amylopectine, peut être quantifiée par l'augmentation de la coloration bleue provoquée, en présence d'iode, par la libération d'amylose à partir d'amylopectine.

La mesure de l'activité enzymatique débranchante est réalisée selon le protocole suivant. 0,4 ml d'une solution d'amylopectine à 1 % contenant un tampon acétate 50 mM à pH 4,5 est incubée à 60 °C pendant 10 minutes. La réaction est démarrée par l'addition de 0,2 ml de pullulanase et elle est arrêtée après 30 minutes par addition de 0,4 ml d'HCl 0,3 M. 0,8 ml d'une solution d'iode à 0,0025 % (v/v) est ensuite ajoutée à 0,2 ml de ce mélange réactionnel et la densité optique est mesurée à 565 nm.

En vue de purifier la pullulanase, on diafiltre la solution aqueuse concentrée de pullulanase par 6 fois 500 ml d'une solution aqueuse de NaCl à 9 g/l et on ajuste le pH de la solution aqueuse, ainsi obtenue, à pH 3,5 par addition d'HCl 25 % (v/v) à température ambiante. La diafiltration consiste à mélanger la solution de pullulanase avec la solution de NaCl, puis à ultrafiltrer la solution obtenue.

Le précipité obtenu est éliminé par centrifugation (5000 tours par minute pendant 30 minutes, BECKMAN JA-10), on récupère le surnageant de centrifugation. Le pH de ce surnageant est ajusté à pH 6,0 par addition de NaOH 5 M. Le précipité obtenu est éliminé par centrifugation.

On récupère le surnageant de centrifugation, que l'on chauffe jusqu'à 55 °C pendant 15 minutes.

Le précipité formé est à nouveau éliminé par centrifugation (5000 tours par minute pendant 30 minutes, BECKMAN JA-10). On récupère le surnageant de centrifugation.

On ajoute à ce surnageant de l'acétone à une concentration finale de 60 % (v/v), la suspension formée est mise à 4 °C durant 2 heures. Le précipité formé à 4 °C est mis en solution dans un tampon MES (acide 2(N-morpholino)éthanesulfonique) 20 mM, CaCl₂ 1 mM (pH 6,0). Cette solution de pullulanase est nommée solution A.

On concentre de nouveau cette solution A par chromatographie d'échange ionique en vue de la purifier. Une colonne d'environ 20 ml de volume interne, vendue sous la marque S-SEPHAROSE® HP HI LOAD 16/10, est préalablement équilibrée par un tampon CH₃COONa 50 mM, NaCl 100 mM (pH 4,0) à un débit de 5 ml/minute. La solution A est diluée 10 fois dans le tampon acétate et 15 ml de cette solution diluée sont déposés sur la colonne. Une phase isocratique est assurée par élution de 80 ml du tampon acétate (100 mM NaCl), suivie d'une élution par 200 ml du tampon acétate 50 mM (pH = 4,0) contenant un gradient linéaire en NaCl (100 - 500 mM).

L'activité pullulanase est mesurée dans chaque fraction.

Les fractions les plus actives sont rassemblées en une solution nommée B (12 ml contenant 0,025 mg/ml de protéines et ayant une activité pullulanase de 0,7 PUN/ml).

A partir de cette solution B on effectue une précipitation par de l'acétone à une concentration finale de 80 % (v/v). Le précipité obtenu est mis en solution dans une volume de 0,6 ml du tampon MES 20 mM, CaCl₂ 1 mM (pH 6,0).

Cette solution de pullulanase est nommée solution C.

La solution C a une teneur en protéines de 0,4 mg/ml, une activité enzymatique de 12 PUN/ml et une activité spécifique de 30 PUN/mg.

Les résultats sont rassemblés dans le tableau 1.

**TABLEAU 1**

| Fractions | Volume | Protéines | | | Activité pullulanase | | | Activité spécifique PUN/mg |
|---|---|---|---|---|---|---|---|---|
| | ml | mg/ml | Total | % | PUN/ml | Total | % | |
| Solution A | 1,5 | 6,48 | 9,7 | 100 | 17,5 | 26,3 | 100 | 2,7 |
| Solution B | 12 | 0,025 | 0,3 | 3 | 0,7 | 8,4 | 32 | 28 |

Le tableau 1 montre que cette étape de la purification a augmenté d'un facteur 10 l'activité spécifique pullulanase de la solution enzymatique.

L'activité débranchante, c'est-à-dire l'activité d'hydrolyse des liens alpha-1,6 dans l'amylopectine, de la pullulanase a également été mesurée comme décrit ci-dessus, par coloration à l'iode après hydrolyse de l'amylopectine. Les résultats montrent que l'activité débranchante a également été enrichie.

### Exemple 3

### Détermination du poids moléculaire

On effectue sur la solution C, telle qu'obtenue à l'exemple 2, une précipitation au moyen d'acide trichloroacétique (10 % (v/v) final). Le précipité obtenu est repris dans un tampon composé de 10 mM TRIS/HCl (pH=8,0), 1mM EDTA, 2,5 % (w/v) SDS (sodium dodécyl sulfate), 5 % (v/v) β-mercaptoéthanol et 0,01 % (p/v) de bleu de bromophenol.

4 µl du précipité repris dans le tampon sont déposés sur un gel de polyacrylamide. Le système de gel utilisé est le système PHASTSYSTEM de PHARMACIA LKB BIOTECHNOLOGY, avec des gels contenant un gradient de polyacrylamide de 10-15 % (v/v) en présence de SDS. Les conditions d'électrophorèse sont celles prescrites par le fournisseur. Une coloration du gel au bleu de Coomassie fait apparaître un polypeptide d'un poids moléculaire d'environ 105 KDaltons, qui est le composant majeur de la solution C.

Ceci est confirmé par l'estimation faite à partir de la séquence en acides aminés de la forme mature de la pullulanase (sans la séquence signal), comme décrit à l'exemple 4, on déduit par calcul un poids moléculaire de 102 KDaltons.

### Exemple 4

### 1. Détermination de la séquence N-terminale

A partir du gel décrit à l'exemple 3, on identifie la séquence N-terminale de la pullulanase en suivant la technique décrite par Bauw et al., (1987), Proc. Natl. Acad. Sci. USA, 84, p. 4806-4810.

Cette séquence (SEQ ID NO:1), ainsi determinée, est la suivante dans le sens amino-carboxy et de gauche à droite :

Asp Gly Asn Thr Thr Thr Ile Ile Val His Tyr Phe Cys Pro Ala Gly

Asp Tyr Gln Pro

### 2. Détermination de la séquence en acides aminés de la pullulanase

La séquence nucléotidique (SEQ ID NO:8) du fragment BamHI-PstI d'environ 4,5 Kb du plasmide pUBCDEBRA11, contenant le gène codant pour la pullulanase, tel qu'obtenu à l'exemple 21, a été déterminée par la méthode de terminaison de chaînes utilisant des dideoxy-nucléotides, de SANGER et al. (1977) Proc. Natl. Acad. Sci. USA 74, p. 5463-5467.

Les oligonucléotides synthétiques utilisés pour initier les réactions d'élongation par la T7 DNA polymérase ont été synthétisés par la méthode de BEAUCAGE et al. (1981) Tetrahedron letters 22, p. 1859-1882. Le séquençage a été effectué suivant le protocole donné par le fournisseur du kit de séquençage (PHARMACIA), en procédant à une dénaturation de l'ADN double brin par traitement avec du NaOH.

La stratégie de séquençage est décrite par SAMBROOK, 1989, p 13.15 et 13.17. Les gels de polyacrylamide pour le séquençage ont été effectués suivant la technique décrite par SAMBROOK, 1989, p 13.45-13.58.

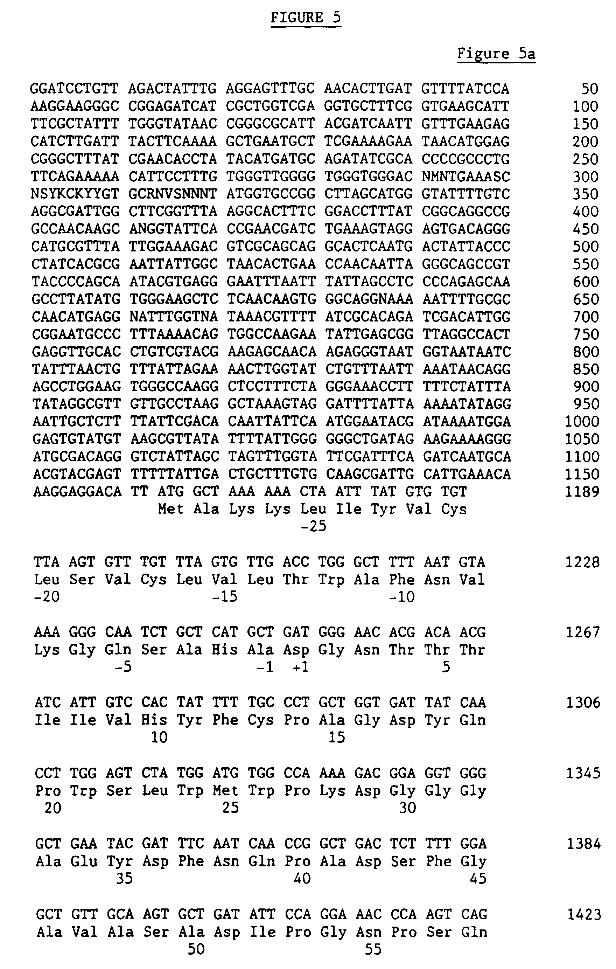

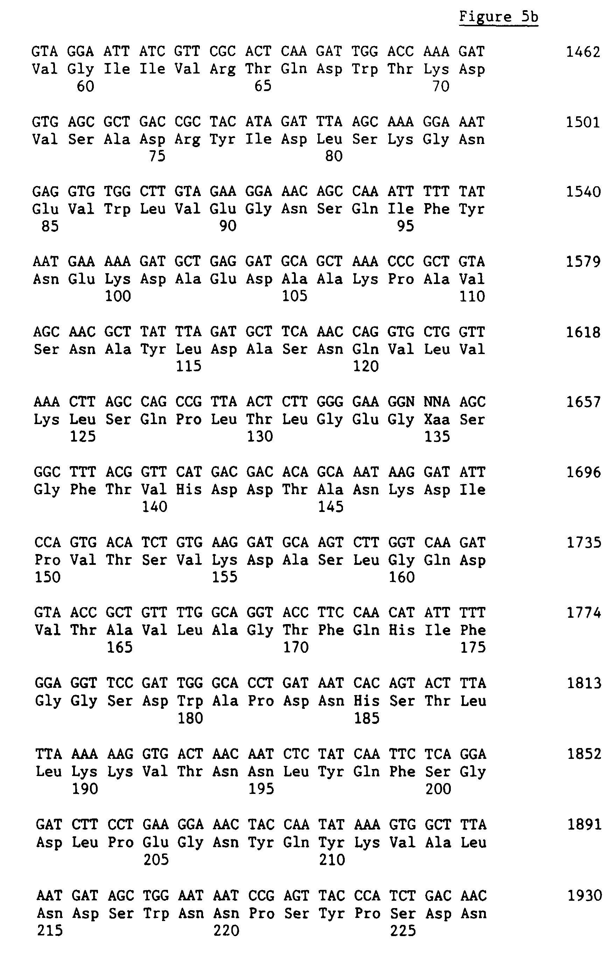

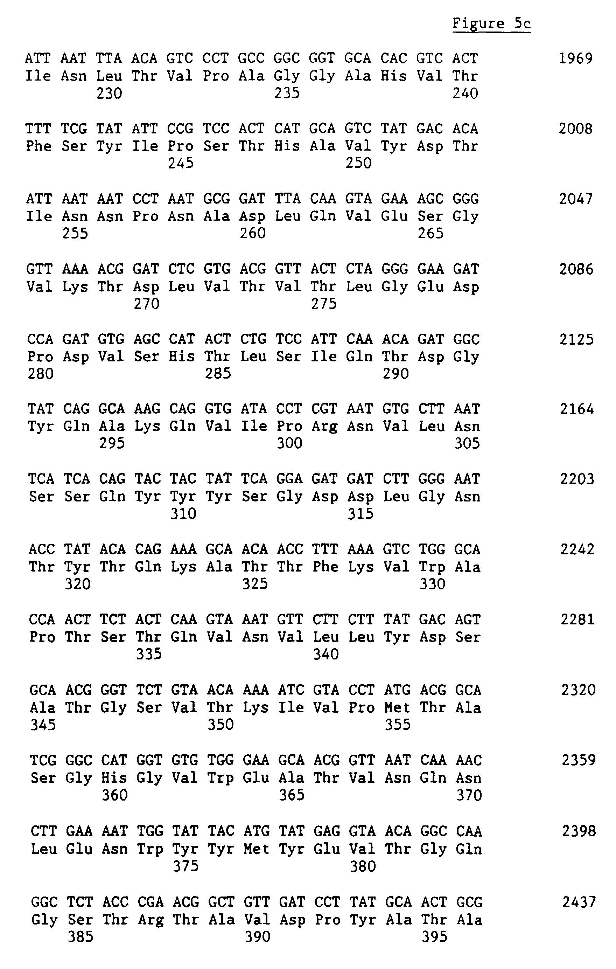

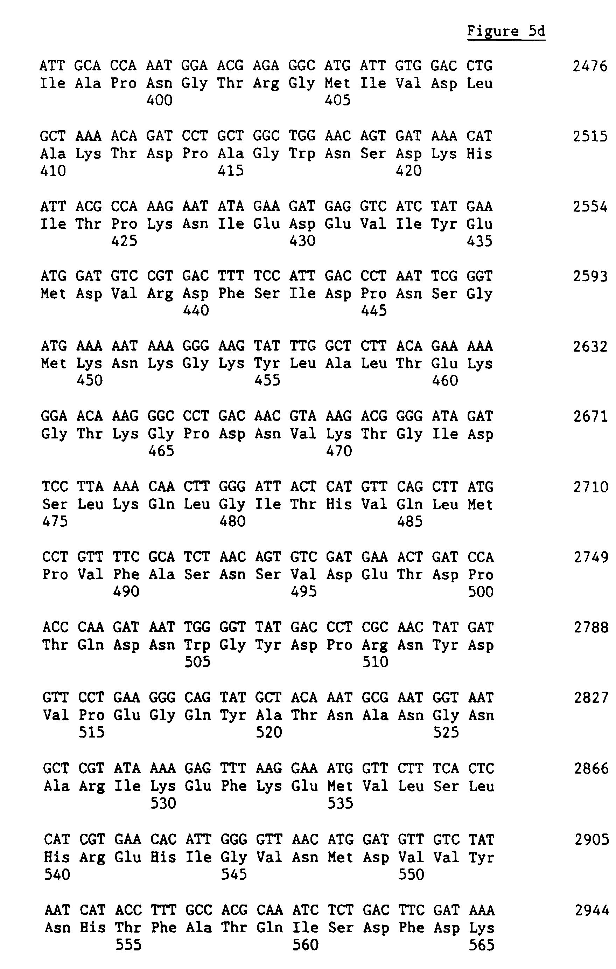

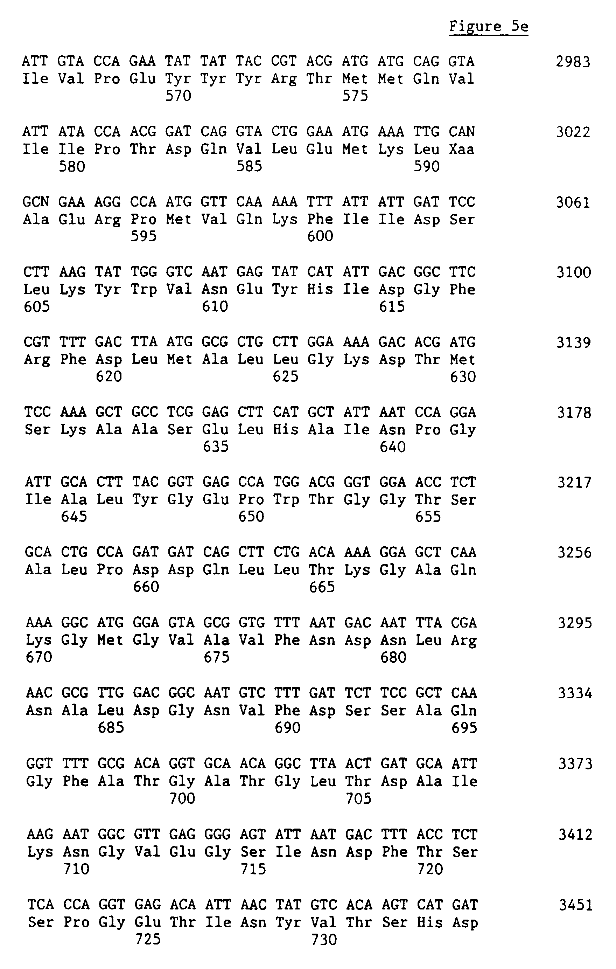

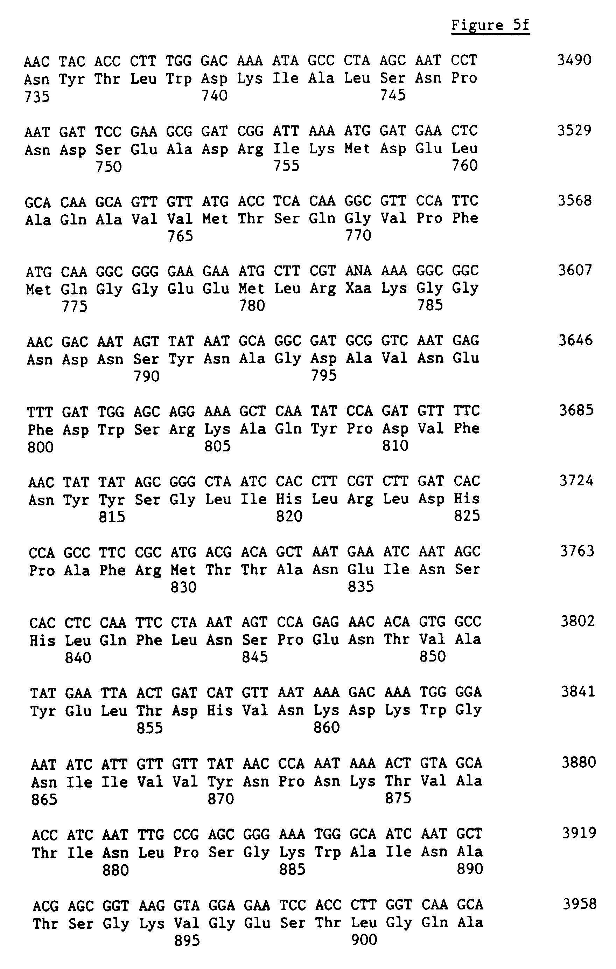

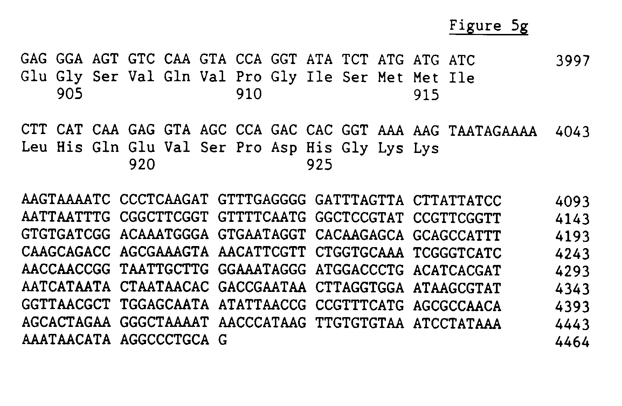

La séquence nucléotidique (SEQ ID NO:8) du fragment d'ADN, depuis le site BamHI jusqu'au site PstI de pUBCDEBRA11, ainsi que la traduction en acides aminés (SEQ ID NO:9) des séquences signal et mature de la pullulanase, a été identifiée (figure 5). Les nucléotides qui n'ont pas été déterminés avec certitude, ont été représentés par le symbole N.

L'analyse de cette séquence montre la présence d'une phase ouverte de lecture qui code pour la pullulanase. On identifie la séquence en nucléotides codant pour la pullulanase mature (SEQ ID NO:10). On déduit ainsi la séquence en acides aminés de la pullulanase mature (SEQ ID NO:11), par traduction de cette phase ouverte de lecture. La figure 4 représente la séquence de nucléotides codant pour la pullulanase mature ainsi que sa traduction en acides aminés.

On vérifie que la séquence N-terminale, déterminée expérimentalement à partir de la protéine tel que décrit ci-dessus, correspond à celle traduite à partir de la séquence d'ADN.

Ceci montre que la pullulanase est synthétisée sous forme d'un précurseur contenant une séquence additionnelle de 29 acides aminés (préséquence). Cette séquence de 29 acides aminés est identifiée (SEQ ID NO:12), ainsi que la séquence de nucléotides (SEQ ID NO:13) correspondante. Cette séquence additionnelle montre les caractéristiques typiques d'une séquence signal de sécrétion qui est éliminée lors de l'exportation de l'enzyme vers l'extérieur de la cellule (Freudl, (1992), Journal of Biotechnology, 23, p. 231-240).

Cette séquence de 29 acides aminés est la suivante :

ATG GCT AAA AAA CTA ATT TAT GTG TGT
Met Ala Lys Lys Leu Ile Tyr Val Cys
-25

TTA AGT GTT TGT TTA GTG TTG ACC TGG GCT TTT AAT GTA
Leu Ser Val Cys Leu Val Leu Thr Trp Ala Phe Asn Val
-20 -15 -10

AAA GGG CAA TCT GCT CAT GCT
Lys Gly Gln Ser Ala His Ala
-5 -1

### Exemple 5

### Distribution en acides aminés

La distribution en acides aminés de la pullulanase mature, déterminée à partir de la séquence en acides aminés de la pullulanase (exemple 4), est résumée dans le tableau 2.

**TABLEAU 2**

| Symbole | Acides aminés | Nombre | % (en poids moléculaire) |
|---|---|---|---|
| D | acide aspartique | 75 | 8,5 |
| N | asparagine | 69 | 7,7 |
| V | valine | 72 | 7,0 |
| T | thréonine | 70 | 6,9 |
| Y | tyrosine | 42 | 6,7 |
| L | leucine | 60 | 6,7 |
| K | lysine | 48 | 6,0 |
| S | serine | 64 | 5,5 |
| I | isoleucine | 47 | 5,2 |
| E | acide glutamique | 40 | 5,1 |
| Q | glutamine | 39 | 4,9 |
| A | alanine | 69 | 4,8 |
| P | proline | 46 | 4,4 |
| G | glycine | 75 | 4,2 |
| F | phénylalanine | 27 | 3,9 |
| W | tryptophane | 18 | 3,3 |
| M | méthionine | 23 | 3,0 |
| H | histidine | 22 | 3,0 |
| R | arginine | 18 | 2,8 |
| X | inconnu | 3 | 0,3 |
| C | cystéine | 1 | 0,1 |

### Exemple 6

### Détermination du point isoélectrique

On effectue sur la solution C, telle qu'obtenue à l'exemple 2, une électrophorèse IEF (iso electro focusing) dans un gradient de pH variant de 4,0 à 6,5.

On dépose un volume correspondant à 0,12 unités de pullulanase en triple sur gel. Après migration, un tiers du gel est coloré au bleu de Coomassie.

Les deux autres parties du gel sont recouvertes par des gels d'agar (1 % poids/volume) tamponnés par CH₃COONa 100 mM, CaCl₂ 1 mM, MgCl₂ 1 mM (pH 4,5) contenant respectivement 0,1 % (p/v) d'AZCL-pullulane ou 1 % (p/v) d'amylopectine. L'ensemble (gel d'acrylamide-gel d'agar) ainsi obtenu est ensuite incubé à 60 °C en atmosphère saturée en humidité durant 16 heures. Le gel recouvert de la surcouche d'amylopectine est ensuite incubé à température ambiante dans une solution contenant de 3 mM I₂, 50 mM KI afin de révéler l'activité débranchante par apparition de la coloration bleue.

La révélation à l'iode du gel amylopectine révèle un halo bleu foncé, signe d'une activité débranchante, à un point isoélectrique compris entre environ 4,1 et environ 4,5 pour l'enzyme de la présente invention. La révélation de l'activité pullulanase indique le même résultat.

Ceci démontre que la pullulanase de la présente invention présente une activité pullulanase et une activité débranchante.

Ceci démontre que la pullulanase de la présente invention est capable d'hydrolyser les liens de type α-1,6, aussi bien dans le pullulane que dans l'amylopectine. Ceci démontre une spécificité faible de la pullulanase de la présente invention vis-à-vis de son substrat.

Ceci est confirmé par l'estimation faite à partir de la séquence en acides aminés de la forme mature de la pullulanase (sans la séquence signal), comme décrit à l'exemple 4, on déduit par calcul un point isoélectrique de 4,5.

### Exemple 7

### Profil d'activité en fonction du pH et de la température pour la pullulanase produite par la souche naturelle (Bacillus deramificans)

On mesure l'activité enzymatique de la pullulanase à différentes températures (55, 60 et 65 °C) et à différents pH (de 3,25 à 7) en tampon citrate - phosphate 50 mM par la mesure des sucres réducteurs libérés. On met en oeuvre la solution C de pullulanase, telle qu'obtenue à l'exemple 2, diluée à environ 1 PUN/ml.

Les résultats sont rassemblés dans le tableau 3.

Au cours de cet essai l'activité enzymatique maximale a été mesurée, par la mesure des sucres réducteurs libérés, pour l'échantillon placé à un pH d'environ 4,3 et à une température d'environ 60 °C durant 15 minutes. Par définition on a donc attribué à cet échantillon une activité enzymatique relative de 100 %.

Cet exemple montre que la pullulanase selon l'invention présente une activité enzymatique optimale mesurée à une température d'environ 60 °C dans une gamme de pH comprise entre 4,0 et 4,8.

Cet exemple montre également que la pullulanase selon l'invention présente une activité enzymatique optimale, mesurée à un pH d'environ 4,3, dans une gamme de température comprise entre 55 et 65 °C.

De plus, cet exemple montre que la pullulanase selon l'invention développe une activité enzymatique de plus de 80 % de l'activité enzymatique maximale dans une gamme de pH compris entre environ 3,8 et environ 4,9.

**TABLEAU 3**

| Activité relative de l'enzyme % | | | |
|---|---|---|---|
| pH | Température °C | | |
| | 55 | 60 | 65 |
| 3,25 | 5,7 | 2,2 | 4,3 |
| 3,75 | 80,8 | 83,7 | 11,5 |
| 4,30 | 87,9 | 100 | 84,1 |
| 4,90 | 82,4 | 87,1 | 68 |
| 5,50 | 50,6 | 39,6 | 13,5 |
| 6,00 | 7,5 | 2,9 | 0 |
| 6,40 | 0 | 0 | 0 |

### Exemple 8

### Stabilité vis-à-vis du pH de la pullulanase produite par la souche naturelle (Bacillus deramificans)

On dilue la solution A de la pullulanase, telle qu'obtenue à l'exemple 2, de telle sorte qu'elle développe une activité enzymatique d'environ 0,7 PUN/ml, dans différents tampons citrate-phosphate 100 mM à des pH variants entre pH 3,0 et 7,0. On incube les différentes solutions diluées contenant la pullulanase pendant 60 minutes à 60 °C.

Ensuite on mesure l'activité enzymatique de ces différentes solutions après incubation pendant 60 minutes à pH 4,2 à 60 °C en présence de 1,6 % (poids/volume) de pullulane. La quantité de maltotriose formée est mesurée par chromatographie HPLC (comme décrit à l'exemple 2). Les résultats sont rassemblés dans le tableau 4.

Au cours de cet essai l'activité enzymatique maximale a été mesurée, pour l'échantillon placé à un pH d'environ 4,5 et à une température d'environ 60 °C. Par définition on a donc attribué à cet échantillon une activité enzymatique relative de 100 %.

Cet exemple montre que la pullulanase selon l'invention est stable dans une large gamme de pH acide, en effet elle présente une activité enzymatique relative d'au moins 85 % mesurée après une incubation de 60 minutes à une température d'environ 60 °C en l'absence de substrat et dans une gamme de pH compris entre environ 3,5 et environ 5,8. Cet exemple montre également qu'elle présente une activité enzymatique relative supérieure à 90 % mesurée dans une gamme de pH compris entre environ 3,8 et environ 5 sous ces mêmes conditions et qu'elle n'est inactivée qu'à un pH inférieur ou égale à 3 ou supérieur ou égale à 7.

**TABLEAU 4**

| pH | Activité relative % |
|---|---|
| 3 | 0 |
| 3,5 | 90 |
| 4 | 98 |
| 4,5 | 100 |
| 5 | 96 |
| 5,5 | 92 |
| 6 | 89 |
| 6,5 | 75 |
| 7 | 0 |

### Exemple 9

### Détermination de la demi-vie de la pullulanase produite par la souche naturelle (Bacillus deramificans)

On dilue la solution C de la pullulanase, telle qu'obtenue à l'exemple 2, de telle sorte qu'elle développe une activité enzymatique d'environ 0,7 PUN/ml dans un tampon acétate de sodium 100 mM à un pH de 4,5. On incube la solution diluée contenant la pullulanase à 60 °C et des échantillons sont prélevés à des temps différents.

Une mesure de l'activité enzymatique par la méthode des sucres réducteurs (méthode de SOMOGYI décrite ci-dessus) est ensuite réalisée.

Au cours de cet essai l'activité enzymatique maximale a été mesurée pour l'échantillon au temps 0. Par définition on a donc attribué à cet échantillon une activité enzymatique relative de 100 %.

Les résultats sont rassemblés dans le tableau 5.

**TABLEAU 5**

| Temps heures | Activité relative % |
|---|---|
| 0 | 100 |
| 16 | 76 |
| 24 | 74 |
| 40 | 57 |
| 48 | 54 |
| 64 | 47 |

Cet exemple montre que la pullulanase est thermostable à pH acide.

Cet exemple montre que la demi-vie de la pullulanase est d'environ 55 heures dans ces conditions. En effet la pullulanase présente une activité enzymatique relative d'au moins 50 % mesurée après une incubation de 55 heures à une température d'environ 60 °C dans une solution tamponnée à un pH d'environ 4,5 et en l'absence de substrat.

Cet exemple montre de plus que la pullulanase selon l'invention présente une activité enzymatique relative d'au moins 55 % mesurée après une incubation de 40 heures à une température d'environ 60 °C dans une solution tamponnée à un pH d'environ 4,5 et en l'absence de substrat. Cet exemple montre également qu'elle présente une activité enzymatique relative d'au moins 70 % mesurée après une incubation de 24 heures sous ces mêmes conditions.

### Exemple 10 et exemple 11R (de comparaison)

### Saccharification

On prépare un milieu de saccharification en mettant en suspension, dans de l'eau, de l'amidon de maïs à une concentration de 35 % (poids/poids) en poids de matières sèches d'amidon et du chlorure de calcium à une concentration de 0,02 % (poids/volume).

On liquéfie cette suspension d'amidon de maïs en présence d'α-amylase, vendue sous la marque TAKATHERM® L-340 par SOLVAY ENZYMES, à 105 °C pendant 5 minutes à pH 6,0.

On refroidit rapidement l'amidon liquéfié, ainsi obtenu, à une température de 95 °C et on continue l'hydrolyse pendant 120 minutes à 95 °C sous agitation. A ce stade le degré d'hydrolyse est compris entre 10 et 12 DE (DE représente l'unité de "Dextrose Equivalents", c'est-à-dire le nombre d'extrémités réductrices exprimées en équivalent glucose).

L'amidon liquéfié ainsi obtenu est dilué à une concentration finale de 32 g de poids sec par 100 g de milieu de saccharification.

On refroidit le milieu de saccharification obtenu à une température de 60 °C.

On ajuste le pH de ce milieu de saccharification à différentes valeurs avec de l'acide acétique, depuis 3,9 jusqu'à 4,8 et on le maintient constant au cours de la saccharification.

On ajoute au milieu de saccharification une quantité de glucoamylase correspondant à 0,176 DU/g.ds. (unité enzymatique glucoamylase par g de matières sèches de milieu de saccharification), la glucoamylase utilisée est vendue sous la marque DIAZYME L-200 par SOLVAY ENZYMES.

Pour l'exemple 10 selon l'invention, on ajoute également au milieu de saccharification une quantité de pullulanase, correspondant à 0,075 PUN/g.ds., sous forme de solution aqueuse concentrée de pullulanase (solution A), telle que décrite à l'exemple 2.

L'exemple 11R de comparaison est réalisé, comme décrit ci-dessus pour l'exemple 10, mais sans addition de pullulanase.

Après 48 heures, on arrête la saccharification et on analyse les produits obtenus par chromatographie (comme décrit à l'exemple 2).

Les résultats sont rassemblés dans le tableau 6.

Cet exemple montre que la pullulanase de l'invention est efficace en saccharification. La pullulanase de l'invention possède donc toutes les propriétés adéquates compatibles avec les conditions industrielles réelles de saccharification de l'amidon.

Cet exemple montre que le taux de conversion de l'amidon est supérieur en présence de la pullulanase selon l'invention, à différents pH, et ceci jusqu'à un pH très acide, c'est-à-dire au moins 3,9.

**TABLEAU 6**

| pH | Exemples | Produits obtenus en % | | | |
|---|---|---|---|---|---|
| | | Glucose | DP2 | DP3 | > DP3 |
| 3,9 | 11R | 94,18 | 2,92 | 0,54 | 2,37 |
| | 10 | 95,63 | 2,90 | 0,73 | 0,73 |
| 4,2 | 11R | 94,18 | 2,98 | 0,56 | 2,29 |
| | 10 | 94,79 | 4,30 | 0,56 | 0,38 |
| 4,5 | 11R | 93,72 | 2,88 | 0,57 | 2,83 |
| | 10 | 95,49 | 3,00 | 0,75 | 0,76 |
| 4,8 | 11R | 93,32 | 2,79 | 0,60 | 3,30 |
| | 10 | 95,25 | 2,70 | 0,87 | 1,18 |

DP2 représente les oligosaccharides contenant deux unités de glucose (dimère de glucose), DP3 les oligosaccharides contenant trois unités de glucose (trimère de glucose), >DP3 les oligosaccharides contenant plus de 3 unités de glucose.

### Exemple 12 et exemple 13R (de comparaison)

### Saccharification

On répète l'exemple 10, mais en fixant le pH de milieu de saccharification à un pH de 4,2.

On ajoute au milieu de saccharification une quantité de glucoamylase correspondant à 0,17 DU/g.ds. (unité enzymatique par g de matières sèches de milieu de saccharification), la glucoamylase utilisée est vendue sous la marque DIAZYME L-200 par SOLVAY ENZYMES.

Pour l'exemple 12 selon l'invention, on ajoute également au milieu de saccharification diverses quantités de pullulanase, correspondant respectivement à 0,0325 PUN/g.ds., 0,050 PUN/g.ds., 0,075 PUN/g.ds. et 0,10 PUN/g.ds. (unité enzymatique pullulanase par gramme de matières sèches de milieu de saccharification), sous forme de solution aqueuse concentrée de pullulanase (solution A), telle que décrite à l'exemple 2.

L'exemple 13R de comparaison est réalisé, comme décrit ci-dessus pour l'exemple 12, mais sans addition de pullulanase.

Les résultats sont rassemblés dans le tableau 7.

Cet exemple montre que la quantité de pullulanase qu'il faut mettre en oeuvre pour observer une augmentation du pourcentage de glucose produit est inférieure à 0,0325 PUN/g.ds.

**TABLEAU 7**

| Exemples | Pullulanase PUN/g.ds. | Produits obtenus en % | | | |
|---|---|---|---|---|---|
| | | Glucose | DP2 | DP3 | >DP3 |
| 13R | 0 | 94,78 | 3,55 | 0,73 | 0,94 |
| 12 | 0,0325 | 95,16 | 3,45 | 0,78 | 0,61 |
| | 0,050 | 95,30 | 3,39 | 0,74 | 0,56 |
| | 0,075 | 95,25 | 3,47 | 0,74 | 0,55 |
| | 0,10 | 95,27 | 3,49 | 0,70 | 0,53 |

### Exemple 14

### Construction du plasmide pUBDEBRA1

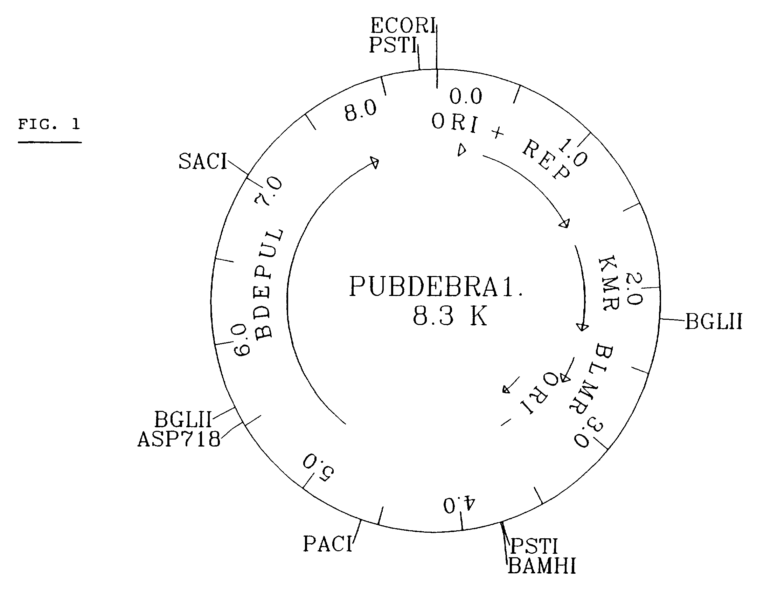

Le plasmide pUBDEBRA1 (figure 1) contient le gène qui code pour la pullulanase de la souche de Bacillus deramificans T 89.117D sous le contrôle de son propre promoteur de transcription, introduit dans le vecteur pUB131. La construction du plasmide pUBDEBRA1 est décrite ci-après.

L'ADN chromosomique est extrait et purifié à partir d'une culture de la souche de Bacillus deramificans T 89.117D (identifié sous le numéro LMG P-13056).

Pour ce faire, une culture de 200 ml de ce bacille est réalisée en milieu liquide MYE (exemple 1).

Lorsque cette culture est réalisée et qu'elle est en phase stationnaire, elle est centrifugée (rotor BECKMAN JA-10) à 5000 tours par minute pendant 10 minutes. Le culot de centrifugation ainsi obtenu est repris dans 9 ml de tampon TRIS-HCl (tris(hydroxyméthyl)aminométhane acidifié avec HCl) 0,1 M, à un pH de 8, EDTA (acide éthylènediaminetétraacétique) 0,1 M, NaCl 0,15 M contenant 18 mg de lysozyme, la suspension ainsi obtenue est incubée 15 minutes à 37 °C.

Le lysat ainsi obtenu est ensuite traité par 200 µl d'une solution de RNAse à 10 mg/ml pendant 20 minutes à 50 °C. 1 ml d'une solution de SDS (sodium dodécyl sulfate) à 10 % est alors ajoutée à ce lysat. Ensuite on incube ce lysat durant 30 minutes à 70 °C.

Puis le lysat est refroidi aux environ de 45 °C, on y ajoute ensuite 0,5 ml d'une solution de protéinase K à 20 mg/ml (préparée extemporanément).

Le lysat est incubé à 45 °C sous agitation manuelle jusqu'à l'obtention d'une solution transparente.

On effectue à partir de cette solution transparente plusieurs extractions au phénol sous les conditions et en suivant les procédures décrites dans Molecular Cloning - a laboratory manual - SAMBROOK, FRITSCH, MANIATIS - second edition, 1989, à la page E.3, jusqu'à l'obtention d'une interface propre, comme cela y est décrit.

L'ADN est précipité par 20 ml d'éthanol. Le précipité est récupéré par centrifugation à 5000 tours par minute pendant 5 minutes, puis mis en suspension dans 2 ml de tampon TE à pH 8,0 (10 mM TRIS-HCl, 1mM EDTA à pH 8,0).

L'ADN ainsi obtenu est ensuite clivé partiellement par l'enzyme de restriction Sau3AI. Les conditions de restriction dans cet exemple, et dans tous les autres exemples de cette demande, sont celles décrites par SAMBROOK et al (page 5.28-5.32), à part que ces conditions de restriction sont augmentées d'un facteur 10, afin d'obtenir une quantité d'ADN suffisante pour les étapes de purification suivantes.

Le rapport entre la quantité d'ADN mise en oeuvre et la quantité d'enzyme est ajusté afin d'obtenir un maximum de fragments de taille comprise entre 5 et 10 kpb (kpb : 10³ paires de bases).

L'ensemble des fragments ainsi obtenus est ensuite soumis à une électrophorèse en gel d'agarose (0,8 %) comme décrit par SAMBROOK et al (page 6.01-6.19) et les fragments de taille comprise entre 5 et 10 kpb sont isolés et purifiés par la méthode GENE CLEAN. Ils sont ensuite ligaturés avec le plasmide pBR322 qui est vendu par la société BIOLABS, [CLONTECH LABORATORIES (U.S.A.) catalogue n° 6210-1] coupé au site BamHI et déphosphorylé comme décrit par SAMBROOK et al (page 1.60-1.61). Cette même technique est utilisée dans les autres exemples.

La ligation ainsi obtenue est transformée dans des cellules de E. coli MC1061 [CLONTECH LABORATORIES, catalogue n° C-1070-1] par électroporation (SAMBROOK et al, page 1.75-1.81); les souches transformées sont sélectionnées sur boîte de Pétri contenant le milieu LB (Luria-Bertani) gélosé et 100 µg/ml d'ampicilline, après croissance à 37 °C pendant environ 18 heures. Le milieu LB est décrit par SAMBROOK et al (page A.4). Ce milieu contient 10 g/l de tryptone, 5 g/l d'extrait de levure et 10 g/l de chlorure de sodium.

Les colonies obtenues sur ces boîtes sont ensuite répliquées sur deux boîtes du même milieu.

Une des deux boîtes est recouverte d'un milieu gélosé contenant 1 % (p/v) d'agar, 100 mM d'acétate de sodium (pH 4,5) et 0,1 % (p/v) d'AZCL-pullulane. Après incubation à 60 °C durant 18 heures, la colonie montrant une zone d'hydrolyse de l'AZCL-pullulane la plus importante est repérée, et la colonie correspondante est isolée sur l'autre boîte répliquée.

On obtient ainsi une souche dont on extrait le plasmide qui est dénommé pBRDEBRA3. Le fragment EcoRI-BamHI d'environ 4,6 Kpb du plasmide pBRDEBRA3 est obtenu par une double digestion du plasmide pBRDEBRA3 avec BamHI et EcoRI, puis une purification par électrophorèse en gel d'agarose (0,8 % p/v). Ce fragment est ensuite ligaturé avec le vecteur pUB131, (décrit dans la demande de brevet européen 0 415 296), qui a préalablement fait l'objet d'une double digestion avec BamHI et EcoRI, aux sites BamHI et EcoRI, en utilisant la souche de Bacillus subtilis PSL1 comme hôte.

La souche de Bacillus subtilis PSL1 peut être obtenue à la collection B.G.S.C. sous le numéro 1A510 (BACILLUS GENETIC STOCK CENTER, Ohio State University, Etats-Unis).

Le plasmide pUBDEBRA1, ainsi obtenu, est isolé et purifié à partir des cellules transformées PSL1 par la technique de la lyse alcaline (SAMBROOK et al, page 1.25-1.28). Cette même technique est utilisée dans les autres exemples.

Toutes les souches de Bacillus subtilis transformées sont capables d'exprimer le gène de la pullulanase et de sécréter la pullulanase.

Les souches PSL1 transformées contenant le plasmide pUBDEBRA1 sont repiquées sur une boîte de Pétri contenant le milieu LB avec 25 µg/ml de kanamycine.

Les colonies obtenues sont recouvertes d'une surcouche d'agarose (1 % poids/volume) contenant de l'AZCL-pullulane (0,1 % poids/volume) et de l'acétate de sodium (100 mM, pH 4,5). Après une incubation à 60 °C durant 18 heures, on observe que toutes les colonies des souches transformées sont entourées d'un halo d'hydrolyse de l'AZCL-pullulane.

### Exemple 15

### Préparation de la souche de Bacillus licheniformis SE2 delap1. Identification des parties terminales du gène de la protéase alcaline de la souche hôte de Bacillus licheniformis SE2.

Cet exemple concerne l'identification des parties terminales du gène de la protéase alcaline de la souche hôte de Bacillus licheniformis, afin de préparer le plasmide de délétion pour déléter ledit gène du Bacillus licheniformis SE2.

### 1. Extraction de l'ADN chromosomique à partir de B. licheniformis SE2.

En vue d'isoler le gène de la protéase alcaline de l'ADN chromosomique du Bacillus licheniformis SE2, on extrait d'abord l'ADN chromosomique, en suivant la méthode décrite à l'exemple 14 pour l'extraction de l'ADN chromosomique, à l'exception du milieu de culture constitué du milieu LB, et on le purifie.

### 2. Identification de la partie C-terminale du gène de la protéase alcaline.

L'ADN chromosomique extrait est soumis à une analyse par restriction, analyse décrite dans Molecular Cloning - SAMBROOK et al. (page 1.85) et Molecular Cloning, a laboratory Manual. MANIATIS et al, 1982 Cold Spring Harbor Laboratory, pages 374-379. Les fragments d'ADN obtenus à partir de ces digestions sont séparés selon leur taille sur un gel d'agarose à 0,8 % (poids/volume).

Le gel d'agarose est alors soumis à une analyse par la technique du SOUTHERN BLOT (Technique décrite par SAMBROOK et al - page 9.31), en vue d'identifier les fragments qui contiennent les séquences de nucléotides de la partie C-terminale du gène de la protéase alcaline.

La sonde construite qui est utilisée pour les hybridations, est un oligonucléotide synthétique correspondant à la partie C-terminale du gène de la protéase alcaline. La technique utilisée pour construire l'oligonucléotide synthétique est décrite dans BEAUCAGE, S.L. et al. (1981), Tetrahedron Letters, 22:1859-1882 et en utilisant des β-cyanoéthyl phosphoramidites dans un appareil de BIOSEARCH CYCLONE SYNTHESIZER. La séquence de l'oligonucléotide synthétique qui a été construit est la suivante (SEQ ID NO:2) :

5′ - GGCGGAGCAAGCTTTGTGG - 3′

Ces résultats montrent que la partie C-terminale du gène de la protéase alcaline est localisée sur le fragment PstI d'environ 2,7 kpb.

L'hybridation avec les sondes d'ADN est effectuée en suivant la technique décrite dans Molecular Cloning - SAMBROOK et al. - page 9.52-9.55. Cette même technique est utilisée dans les autres exemples.

La préparation d'ADN chromosomique extrait provenant de la souche de Bacillus licheniformis SE2 est ensuite digérée avec l'enzyme PstI et les fragments obtenus sont séparés selon leur taille par électrophorèse de gel d'agarose (0,8 %).

Les fragments PstI obtenus d'environ 2,7 kpb sont extraits des gels et purifiés par la technique dite "GENE CLEAN" mettant en oeuvre des billes de verre et mise sur le marché par la Société BI0101 (U.S.A.).

Les fragments PstI de 2,7 kpb sont ensuite ligaturés (SAMBROOK et al, page 1.68-1.69) avec le plasmide pUC18 (CLONTECH Laboratories, n° 6110-1), qui a été préalablement digéré au site PstI et déphosphorylé. La ligation ainsi obtenue a été ensuite transformée dans des cellules d'Escherichia coli MC1061 par la technique au CaCl₂ (SAMBROOK et al- page 1.82-1.84). La technique permettant de déphosphoryler les fragments d'ADN ou de linéariser les vecteurs est décrite par SAMBROOK et al (page 1.60-1.61). La technique de la ligation est également décrite par SAMBROOK et al (page 1.68-1.69).

Les souches transformées sont sélectionnées sur boîtes de Pétri contenant le milieu LB gélosé et supplémenté de 100 µg/ml d'ampicilline. Les souches transformées à partir d'E. coli MC1061 ainsi obtenues sont ensuite sélectionnées par hybridation avec l'oligonucléotide synthétique marqué en utilisant la sonde C-terminale utilisée dans l'étude du SOUTHERN et le plasmide pKC1 est isolé.

L'oligonucléotide synthétique est marqué par phosphorylation avec de l'ATP [γ^{-32p}] en utilisant la T4-polynucléotide kinase du phage T4 et en suivant la technique décrite par SAMBROOK et al (page 11.31-11.33).

### 3. Identification de la partie N-terminale du gène de la protéase alcaline.

L'ADN chromosomique extrait est soumis à une analyse par restriction. Les fragments d'ADN obtenus à partir de ces digestions sont séparés selon leur taille sur un gel d'agarose à 0,8 %.

Le gel d'agarose est alors soumis à une analyse par la technique du SOUTHERN BLOT, en vue d'identifier les fragments qui contiennent les séquences de nucléotides de la partie N-terminale du gène de la protéase alcaline.

La sonde qui est utilisée pour les hybridations, est un oligonucléotide synthétique correspondant à la partie N-terminale du gène de la protéase alcaline. La séquence de l'oligonucléotide synthétique qui a été construite est la suivante (SEQ ID NO:3) :

5′ - ATGGCTCCTGGCGCAGGC - 3′

Ces résultats montrent que la partie N-terminale du gène de la protéase alcaline est localisée sur le fragment PstI d'environ 5,5 kpb et également sur un fragment BclI-PstI, plus petit, d'environ 2 kpb. Ce fragment ne contient pas les sites de restriction XbaI, ClaI, HpaI et SphI.

La préparation d'ADN chromosomique extrait provenant de la souche de Bacillus licheniformis SE2 est ensuite digérée avec l'enzyme PstI et les fragments obtenus sont séparés selon leur taille par électrophorèse de gel d'agarose (0,8 %).

Les fragments obtenus d'environ 5,5 kpb sont extraits des gels et purifiés par la technique dite "GENE CLEAN".

Les fragments PstI de 5,5 kpb ainsi obtenus sont ensuite soumis à une série de digestions avec BclI, XbaI, ClaI, HpaI et SphI. Les fragments d'ADN ainsi produits sont ligaturés avec le plasmide pMK4 (tel que décrit dans SULLIVAN et al., (1984), Gene 29:21-26), qui a été préalablement linéarisé par BamHI et PstI. Le plasmide pMK4 peut être obtenu à la collection B.G.S.C. (Bacillus Genetic Stock Center (Ohio State University) Columbus, Ohio, U.S.A.) sous le numéro 1E29.

Les ligations ainsi obtenues ont été ensuite transformées dans des cellules d'Escherichia coli MC1061 par la technique au CaCl₂.

Les souches transformées sont sélectionnées sur boîtes de Pétri contenant le milieu LB gélosé et supplémenté de 100 µg/ml d'ampicilline. Les souches transformées à partir d'E. coli MC1061 ainsi obtenues sont ensuite sélectionnées par hybridation avec l'oligonucléotide synthétique marqué en utilisant la sonde N-terminale dans l'étude du SOUTHERN et le plasmide pKP1 est isolé.

### Exemple 16

### Séquences de la protéase alcaline

On détermine les séquences des fragments introduits dans les plasmides pKP1 et pKC1 à partir des sites Pst1 jusqu'aux sites SacI, en suivant la technique décrite par SAMBROOK et al (pages 13.15 et 13.17 et figure 13.3B).

### Exemple 17

### Construction du plasmide pLD1

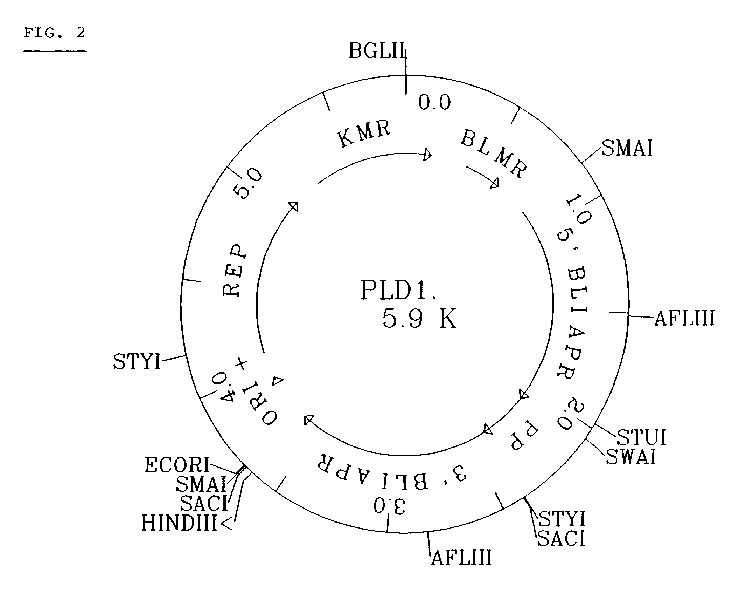

Le plasmide pLD1 (figure 2) est construit dans le but de préparer la souche de Bacillus licheniformis SE2 delap1. La construction du plasmide pLD1 est décrite ci-après.

Le plasmide pKP1 (tel qu'obtenu à l'exemple 15) est instable dans E. coli MC1061. C'est pourquoi le fragment d'ADN chromosomique contenant la partie N-terminale du gène de la protéase alcaline de B. licheniformis SE2 a été introduit dans le vecteur pACYC184 (BIOLABS, U.S.A., sous le numéro #401-M). Cette introduction a été effectuée en introduisant le fragment EcoRI-EcoRI de 1849 bp du plasmide pKP1 dans le site EcoRI du plasmide pACYC184 et la ligation est utilisée pour transformer les cellules d'E. coli MC1061. Le plasmide pKPN11 est ainsi obtenu.

Les souches transformées sont sélectionnées sur boîte de Pétri contenant le milieu LB gélosé et supplémenté de 12,5 µg/ml de tétracycline. L'orientation du fragment EcoRI-EcoRI de 1849 bp dans le plasmide pKPN11 est déterminée par l'analyse de restriction (SAMBROOK et al. - page 1.85 et MANIATIS et al - page 374-379).

Le plasmide pKPN12 est obtenu de la façon suivante : on ôte le fragment StyI-StyI de 1671 bp du plasmide pKPN11 par digestion avec StyI, suivie du remplacement de ce fragment par l'ADN double brin synthétique suivant, qui a été produit préalablement :

5′ - CTTG GAGCTC GTTAAC AGATCT - 3′ (SEQ ID NO:4)

3′ - CTCGAG CAATTG TCTAGA GTTC - 5′ (SEQ ID NO:5)
(StyI) SacI HpaI BalII (StyI)

La digestion des plasmides avec des enzymes de restriction est effectuée en suivant la technique décrite par SAMBROOK et al. - 1989 - chapitres 5.28-5.32.

Le fragment d'ADN venant du plasmide pUB131 qui code pour la résistance à la kanamycine et à la bléomycine ou à la phléomycine a été obtenu comme suit :

Le fragment PstI-TaqI de 2666 bp, qui porte les gènes qui codent pour la résistance à la kanamycine et à la bléomycine ou à la phléomycine, est obtenu par double digestion PstI-TaqI du plasmide pUB131. Ce fragment est introduit dans les sites PstI-AccI du plasmide pBS- (STRATAGENE, U.S.A., sous le numéro 211202). On obtient ainsi le plasmide pBSKMPM.

Durant la préparation du plasmide pBSKMPM, une petite délétion dans la région de la liaison avec le plasmide pBS-apparaît, cela entraîne la perte des sites SphI et PstI dans le plasmide pBSKMPM. Le plasmide pBSKMPM est utilisé pour produire un ADN simple brin mis en oeuvre pour effectuer une mutagénèse dirigée en vue d'introduire les deux nucléotides synthétiques dont les sites SmaI sont identifiés ci-après, l'un est situé en amont et l'autre en aval des gènes de résistance à la kanamycine et à la phléomycine.

La technique de la mutagénèse dirigée est décrite par SAMBROOK et al - page 15.74-15.79. Elle utilise le kit de mutagénèse vendue par BIO-RAD (n° 170-3576).

Les séquences des oligonucléotides synthétiques mis en oeuvre pour la mutagénèse sont les suivantes (respectivement SEQ ID NO:6 et SEQ ID NO:7) :

5′ - CATCTAATCTTCAACACCCGGGCCCGTTTGTTGAAC - 3′
SmaI

5′ - CAAAATAAAAAAGATACAACCCGGGTCTCTCGTATCTTTTAT - 3′
SmaI

Le plasmide obtenu par cette mutagénèse en présence des deux oligonucléotides est le plasmide pBSKMPM1. Ce plasmide contient deux sites de restriction SmaI qui permettent l'isolement du fragment d'ADN contenant les gènes qui codent pour la résistance à la kanamycine et la phléomycine.

Le fragment SmaI-SmaI de 1597 bp du plasmide pBSKMPM1 est ensuite introduit dans le site SmaI du plasmide pKPN12, le plasmide pKPN14 est ainsi obtenu.

La bonne orientation du fragment introduit dans le plasmide pKPN14 est vérifiée en effectuant une sélection sur les préparations d'ADN plasmidique par l'analyse de restriction (SAMBROOK et al - page 1.85).

Le fragment d'ADN, présent sur le plasmide pKC1 et localisé en aval de la séquence N-terminale de la protéase alcaline, est isolé sur le fragment SacI-HindIII de 1,2 kpb du plasmide pKC1 (tel qu'obtenu à l'exemple 15) par digestion, d'abord avec HindIII.

L'extrémité 5' saillante de HindIII est rendue à bouts droits par un traitement avec le fragment Klenow de l'ADN polymérase (SAMBROOK et al- page F.2-F.3). La restriction SacI est alors effectuée en vue de produire le fragment souhaité SacI-HindIII à bouts droits. Ce fragment est introduit dans les sites HpaI et SacI du plasmide pKPN14, en produisant le plasmide pLID1.

Toutes ces constructions sont effectuées par transformation de la souche d'E. coli MC1061 en présence de tétracycline (12 µg/ml) pour la sélection des souches transformées.

Un plasmide capable de se multiplier dans B. subtilis et dans B. licheniformis, est construit à partir du plasmide pLID1 en remplaçant les fonctions de réplication d'E. coli, qui sont portées par le fragment BglII-BglII de 3623 bp du plasmide pLID1, par le fragment qui porte les fonctions de réplication de Bacillus : fragment BglII-BamHI de 2238 bp isolé à partir du plasmide pUB131.

Ce remplacement des fonctions de réplication d'E. coli par celles de Bacillus a été effectué en isolant d'abord le fragment BglII-BglII de 3,6 kpb à partir du plasmide pLID1 par digestion du plasmide pLID1 avec BglII et BamHI. Une digestion BamHI supplémentaire a été nécessaire, en effet une digestion BglII seule résulterait en fragments de taille identique qui ne pourraient pas être séparés par électrophorèse sur gel d'agarose. Le fragment BglII-BglII de 3,6 kpb est alors cloné, dans la souche de Bacillus subtilis SE3, dans le fragment BglII-BamHI de 2238 bp qui a été isolé du plasmide pUB131, en produisant le plasmide pLD1 (figure 2).

La souche de Bacillus subtilis SE3 a été déposée le 21 juin 1993 à la collection nommée BELGIAN COORDINATED COLLECTIONS OF MICROORGANISMS conformément au Traité de Budapest sous le numéro LMG P-14035.

### Exemple 18

### Construction de Bacillus licheniformis SE2 delap1

Les modifications souhaitées dans l'ADN chromosomique de la souche de Bacillus licheniformis SE2 sont effectuées par les techniques basées sur la recombinaison homologue. Les modification sont effectuées pour produire la souche de Bacillus licheniformis SE2 delap1.

Le plasmide pLD1 est transformé dans B. licheniformis SE2 par la technique des protoplastes décrite dans Molecular Biological Methods for Bacillus (pages 150-151) et sous les conditions définies à l'exception des modifications suivantes : la poudre de lysozyme est ajoutée à 5 mg/ml dans le SMMP, au lieu de 1 mg/ml comme définie à l'étape 7 de la procédure décrite, l'incubation pour obtenir une lyse maximale avec le lysozyme est de 60 minutes, la régénération est faite dans le milieu DM3 (décrit par Molecular Biological Methods for Bacillus (Harwood et al, eds) John Wiley and Sons (1990) (pages 150-151) contenant 200 µg/ml de kanamycine.

Une souche transformée est isolée et la carte de restriction du plasmide pLD1, introduit dans cette souche, est vérifiée.

La souche transformée est mise en culture dans 50 ml d'un milieu LB supplémenté de 2 g/l de glucose et de 25 µg/ml de kanamycine, pendant 18 heures à 37 °C.

Un échantillon de culture (0,1 ml) est prélevé et sert à inoculer un erlenmeyer contenant 50 ml de même milieu LB. La culture est incubée à 37 °C pendant 18 heures. Un échantillon de cette culture est prélevé et testé sur boîte de Pétri contenant le milieu LB gélosé et supplémenté de 25 µg/ml de kanamycine et de 1 % (poids/volume) de lait écrémé (DIFCO), pour détecter la présence de protéase.

L'absence de halo d'hydrolyse autour des colonies qui présentent une croissance sur ces boîtes de Pétri indique que ces colonies sont incapables de produire une protéase alcaline.

Les cultures et les tests sont répétés jusqu'à l'obtention d'une souche (apr⁻, Km^{r}), c'est-à-dire à la fois ne produisant plus de protéase alcaline (apr⁻) et résistante à la kanamycine (Km^{r}).

Le plasmide pLD1 présent dans cette souche de Bacillus licheniformis SE2 delap1 est ensuite ôté de celle-ci par culture sur un milieu de croissance à 37 °C en absence d'antibiotique.

Cette souche est mise en culture dans 50 ml de milieu LB supplémenté de 2 g/l de glucose, pendant 18 heures à 37 °C. Un volume de 0,1 ml de cette culture est prélevé et sert à inoculer un autre erlenmeyer contenant également 50 ml d'un même milieu, la culture dure 18 heures à 37 °C. Un échantillon est alors prélevé et est étalé sur boîte de Pétri de milieu LB. Les colonies isolées sont repiquées sur une seconde boîte de milieu LB supplémenté de 25 µg/ml de kanamycine. Une souche sensible à la kanamycine (Km^{s}) est isolée. On confirme son phénotype (apr⁻, Km^{s}).

L'ADN chromosomique de cette souche est alors isolé et purifié et la structure de la délétion chromosomique est vérifiée par la technique du SOUTHERN BLOT. Les délétions identifiées sont correctes quant à leur position, ayant eu lieu par une double recombinaison homologue dans les séquences situées en amont (5') et en aval (3') du gène de la protéase alcaline.

Le souche obtenue est appelée B. licheniformis SE2 delap1. Elle ne produit pas de protéase alcaline.

### Exemple 19

### Transformation de Bacillus licheniformis SE2 delap1 avec le vecteur d'expression

Le plasmide pUBDEBRA1 (figure 1), décrit à l'exemple 14, est extrait de son hôte, isolé et purifié (SAMBROOK et al., 1989, p. 1.25-1.28).

On prépare une culture de la souche de B. licheniformis SE2 delap1, décrite à l'exemple 18, puis on transforme cette souche avec ce plasmide selon la technique des protoplastes décrite par MANIATIS et al (pages 150-151).

La souche transformée est sélectionnée sur boîte de Pétri, isolée et purifiée par criblage.

### Exemple 20

### Production de la pullulanase par B. licheniformis SE2 delap1 (pUBDEBRA1)

La souche de B. licheniformis SE2 delap1 transformée par le plasmide pUBDEBRA1, telle qu'obtenue à l'exemple 19, est mise en culture pendant 17 heures à 37 °C dans un milieu de préculture LB supplémenté de 0,5 % (p/v) de glucose et 20 µg/ml de kanamycine. Cette préculture est transférée (5 % (v/v) dans 50 ml de milieu M2 supplémenté de 20 µg/ml de kanamycine. Le milieu M2 contient 30 g de farine de soja, 75 g d'amidon soluble, 2 g de sulfate de sodium, 5 mg de chlorure de magnésium, 3 g de NaH₂PO₄, 0,2 g de CaCl₂.H₂O et 1000 ml d'eau. Le pH de ce milieu M2 est ajusté à 5,8 avec NaOH 10 N avant sa stérilisation. La culture est incubée sous agitation pendant 80 heures à 37 °C. Après 80 heures, la biomasse est éliminée par centrifugation à 5000 tours par minute pendant 10 minutes. Le surnageant de centrifugation est conservé. On mesure l'activité enzymatique sur ce surnageant et on note la présence d'une activité pullulanase.

### Exemple 21

### Construction de Bacillus licheniformis SE2 delap1 (pUBCDEBRA11DNSI) - Intégration chromosomique

Cet exemple concerne l'intégration du gène codant pour la pullulanase dans le chromosome de la souche Bacillus licheniformis SE2 delap1.

Pour ce faire, le fragment EcoRI-BamHI de 4,6 kb du plasmide pBRDEBRA3 est cloné dans les sites EcoRI et BamHI du vecteur pUBC131 par transformation de la souche E. coli MC1061, générant ainsi le plasmide pUBCDEBRA11.

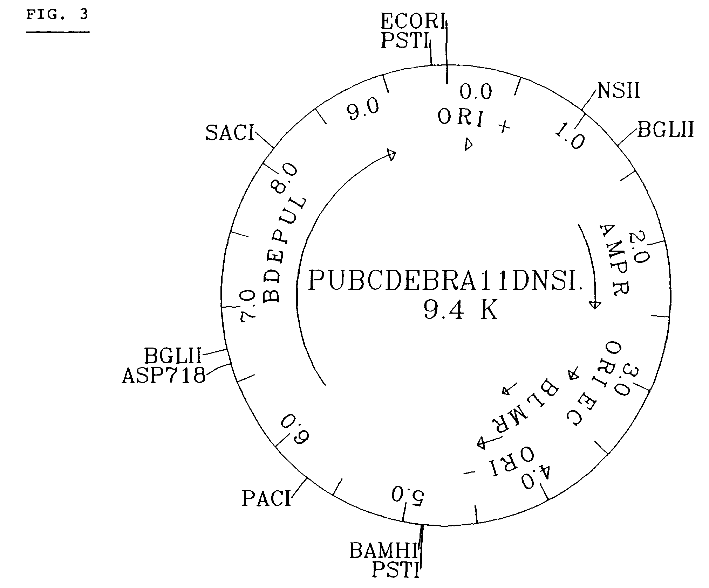

Le vecteur d'intégration pUBCDEBRA11DNSI (figure 3) est ensuite construit en délétant le fragment NsiI-NsiI de 886 bp du plasmide pUBCDEBRA11. Le plasmide ainsi obtenu a perdu la possibilité de se répliquer dans Bacillus, par la perte du fragment NsiI de 886 bp.

Pour effectuer cette construction, le plasmide pUBCDEBRA11 est clivé par l'enzyme de restriction NsiI, et le fragment NsiI-NsiI d'environ 9,4 Kbp est purifié par électrophorèse en gel d'agarose. Ce fragment est ensuite soumis à une ligation afin de le recirculariser. La ligation est transformée dans E. coli MC1061, et le plasmide pUBCDEBRA11DNSI1 est obtenu.

Afin d'intégrer le plasmide pUBCDEBRA11DNSI1 dans la souche B. licheniformis SE2 delap1, il est nécessaire que ce plasmide porte un fragment d'ADN homologue à l'ADN chromosomique. On a donc cloné dans le site BamHI du vecteur d'intégration pUBCDEBRA11DNSI1, des fragments chromosomiques Sau3AI provenant de B. licheniformis.

Pour ce faire, l'ADN chromosomique extrait de la souche Bacillus licheniformis SE2 delap1 est clivé partiellement par l'enzyme de restriction Sau3AI. Les fragments d'ADN de taille comprise entre 1,5 et 3 kb sont ensuite purifiés par gel d'agarose et ligaturés avec le plasmide pUBCDEBRA11DNSI clivé par l'enzyme de restriction BamHI et déphosphorylé. La ligation ainsi obtenue est transformée dans des cellules de MC1061 par électroporation. Après sélection sur milieu LB gélosé contenant 100 µg/ml d'ampicilline, environ 3000 colonies sont obtenues. L'ensemble de ces colonies est mis en suspension dans du milieu LB et une extraction des plasmides est effectuée par la technique de la lyse alcaline (SAMBROOK et al, page 1.25-1.28).

La préparation de plasmides ainsi obtenue est alors introduite par transformation par la technique des protoplastes dans la souche de Bacillus licheniformis SE2 delap1. Les cellules transformées sont sélectionnées sur le milieu de régénération DM3 (décrit dans Molecular Biological Methods for Bacillus (Harwood,C.R. and Cutting, S.M., Eds) J.Wiley and sons, 1990, p. 150-151) pour leur résistance à la phléomycine (17 µg/ml), celle-ci ne pouvant leur être conférée que par intégration chromosomique d'un des plasmides construits ci-dessus.

Les colonies ainsi obtenues sont repiquées sur milieu LB gélosé supplémenté de 5 µg/ml de phléomycine et 0,06 % d'AZCL-pullulane. La colonie présentant le halo d'hydrolyse d'AZCL-pullulane le plus grand est alors isolée et repiquée sur milieu LB gélosé.

Une extraction du contenu plasmidique de cette souche est alors effectuée. La préparation ainsi obtenue est soumise à une analyse par gel d'électrophorèse en agarose, qui montre l'absence de plasmide.

L'ADN chromosomique est extrait et purifié comme décrit à l'exemple 14 et soumis à une analyse par la technique de SOUTHERN, qui montre que le plasmide pUBCDEBRA11DNSI s'est intégré dans l'ADN chromosomique par recombinaison homologue dans un fragment Sau3AI d'environ 3 kb.

Ceci démontre que le gène codant pour la pullulanase de B. deramificans s'exprime dans B. licheniformis, à l'état intégré dans le chromosome.

### Exemple 22

### Procédé de production de la pullulanase par la souche de Bacillus licheniformis SE2 delap1 (pUBCDEBRA11DNSI)

La souche de B. licheniformis SE2 delap1 contenant le gène de la pullulanase sous forme intégrée dans l'ADN chromosomique, telle qu'obtenue à l'exemple 21, est mise en culture pendant 17 heures à 37 °C dans un milieu de préculture LB supplémenté de 0,5 % (p/p) de glucose et de 5 µg/ml de phléomycine. Un volume de 10 ml de cette préculture est inoculé dans 250 ml de milieu M2 (décrit à l'exemple 20), supplémenté de 5 µg/ml de phléomycine, dans des fioles à chicane.

Après 24 heures d'incubation sous agitation à 37 °C, la totalité de la culture ainsi obtenue est introduite dans un fermenteur contenant 6,5 1 de milieu M2. La fermentation est prolongée pendant 72 heures à 37 °C. Le pH est maintenu à une valeur inférieure à 7,0 par addition d'acide phosphorique concentré, le débit d'air est maintenu à 4 litres/minute, et l'agitation est ajustée de façon à obtenir une teneur en oxygène dissous supérieure à 30 % (v/v) de la teneur de saturation.

Après addition de 50 ml d'un agent de floculation, à base de polyamine vendu sous la marque OPTIFLOC® FC 205 par SOLVAY DEUTSCHLAND, à la culture obtenue, la biomasse est éliminée par centrifugation (BECKMAN JA-10) à 5.000 tours/minute pendant 15 minutes et le surnageant obtenu est acidifié à pH 4,5 avec une solution d'HCl 1 M. La solution obtenue est à nouveau centrifugée à 8.000 tours/minute pendant 15 minutes (BECKMAN JA-10).

Le surnageant est ensuite concentré, jusqu'à un volume final de 1 litre, par ultrafiltration en utilisant une unité d'ultrafiltration équipée d'une membrane avec une limite de résolution de 5.000 Daltons.

Puis, on ajoute à cette solution concentrée, de l'acétone à une concentration finale de 60 % (v/v). La suspension formée est incubée à 4 °C pendant 2 heures, puis centrifugée à 8.000 tours/minute pendant 15 minutes (BECKMAN JA-10). Le culot de centrifugation obtenu est mis en suspension dans 100 ml d'une solution aqueuse contenant 30 % (p/v) d'amidon de marque MALTRIN® 250 (GRAIN PROCESSING CORPORATION), 0.3 % (p/v) de benzoate de sodium, et 0.15 % (p/v) de sorbate de potassium, à un pH de 4,5. La préparation purifiée de la pullulanase produite par la souche recombinante, ainsi obtenue, est appelée solution D.

L'activité de la solution D de pullulanase, mesurée par la méthode des sucres réducteurs, est de 150 PUN/ml.

### Exemple 23

### Stabilité de la pullulanase produite par la souche de Bacillus licheniformis SE2 delap1 (pUBCDEBRA11DNSI) vis-à-vis de la température

On dilue la solution D de pullulanase, telle qu'obtenue à l'exemple 22, de telle sorte qu'elle developpe une activité enzymatique comprise entre 10 et 15 PUN/ml, dans un tampon citrate-phosphate 0,05 M à un pH de 4,75.

On repartit cette solution diluée contenant la pullulanase dans 9 tubes à raison de 5 ml de solution diluée par tube.

On incube les différents tubes contenant la solution diluée dans des bain-maries à des températures comprises entre 40 et 80 °C pendant 75 minutes.

Suite à cette incubation, on les place dans un bain de glace pour les refroidir rapidement.

Ensuite on mesure l'activité enzymatique des différentes solutions (conditions de la mesure : température de 60 °C, pH de 4,5, durée d'incubation de 15 minutes).

Au cours de cet essai l'activité enzymatique maximale a été mesurée pour l'échantillon placé à un pH d'environ 4,75 et à une température d'environ 55 °C. Par définition on a donc attribué à cet échantillon une activité enzymatique relative de 100 %.

Les résultats sont rassemblés dans le tableau 12.

**TABLEAU 12**

| Température | Activité enzymatique relative % |
|---|---|
| 40 | 99 |
| 45 | 99 |
| 50 | 100 |
| 55 | 100 |
| 60 | 96 |
| 65 | 83 |
| 70 | 2 |
| 80 | 1 |

Cet exemple montre que la pullulanase selon l'invention présente une activité enzymatique relative d'au moins 80 % mesurée après une incubation de 75 minutes à un pH de 4,75 en l'absence de substrat et dans une gamme de températures inférieures ou égales à 65 °C.

## Revendications

1. Pullulanase, caractérisée en ce que sa séquence N-terminale (SEQ ID NO:1) est la suivante dans le sens amino-carboxy et de gauche à droite :

Asp Gly Asn Thr Thr Thr Ile Ile Val His
1               5                   10

Tyr Phe Cys Pro Ala Gly Asp Tyr Gln Pro
                15                  20

2. Pullulanase selon la revendication 1 caractérisée en ce qu'elle est produite par la souche de Bacillus deramificans.

3. Pullulanase, caractérisée en ce qu'elle comprend la séquence d'acides aminés de 1 à 928 acides aminés, telle qu'illustrée à la figure 4 (SEQ ID NO:11).

4. Pullulanase selon la revendication 3, caractérisée en ce qu'elle est synthétisée sous forme d'un précurseur contenant une séquence additionnelle de 29 acides aminés (SEQ ID NO:12).

5. Pullulanase selon la revendication 1, caractérisée en ce qu'elle développe une activité enzymatique optimale mesurée à une température d'environ 60 °C à un pH d'environ 4,3.

6. Pullulanase selon l'une quelconque des revendications précédentes, caractérisée en ce qu'elle a un poids moléculaire d'environ 100 kDa.

7. Pullulanase selon l'une quelconque des revendications précédentes, caractérisée en ce qu'elle a un point isoélectrique compris entre 4,1 et 4,5.

8. Pullulanase selon l'une quelconque des revendications précédentes, caractérisée en ce qu'elle développe une activité enzymatique de plus de 80 % de l'activité enzymatique maximale dans une gamme de pH compris entre environ 3,8 et environ 4,9 pour une température d'environ 60 °C, l'activité enzymatique maximale étant mesurée à une température de 60 °C et à un pH de 4,3.

9. Pullulanase selon l'une quelconque des revendications précédentes, caractérisée en ce qu'elle montre une activité enzymatique relative supérieure à 90 Z, mesurée à une température d'environ 60 °C en l'absence de substrat, dans une gamme de pH compris entre environ 3,8 et environ 5.

10. Pullulanase selon l'une quelconque des revendications précédentes, caractérisée en ce qu'elle hydrolyse les liaisons glucosidiques α-1,6 présentes dans l'amylopectine.

11. Pullulanase selon l'une quelconque des revendications précédentes, caractérisée en ce qu'elle dégrade le pullulane en maltotriose.

12. Pullulanase selon l'une quelconque des revendications précédentes, caractérisée en ce qu'elle hydrolyse l'amylopectine en formant des oligosaccharides.

13. Pullulanase selon l'une quelconque des revendications précédentes, caractérisée en ce qu'elle catalyse la réaction de condensation du maltose en formant des tétraholosides.

14. Pullulanase selon l'une quelconque des revendications précédentes, caractérisée en ce qu'elle est susceptible d'être obtenue à partir de la souche de Bacillus deramificans LMG P-13056 ou un dérivé ou mutant de cette souche.

15. Pullulanase selon l'une quelconque des revendications 1 à 13, caractérisée en ce qu'elle est produite, de façon hétérologue, par un microorganisme du genre Bacillus qui contient un gêne codant pour une protéase alcaline lorsqu'il est à l'état sauvage, le gène codant pour la protéase alcaline ayant été délété du microorganisme du genre Bacillus.

16. Procédé pour la production d'une pullulanase selon l'une quelconque des revendications 1 à 14, caractérisé en ce qu'il comprend la culture de la souche de Bacillus deramificans ou d'un dérivé de cette souche capable de produire la pullulanase dans un milieu nutritif approprié contenant des sources de carbone et d'azote et des sels minéraux sous condition d'aérobiose et la récolte de la pullulanase ainsi obtenue.

17. Procédé pour la préparation d'une pullulanase selon l'une quelconque des revendications 1 à 14, caractérisé en ce qu'il comprend l'isolement d'un fragment d'ADN codant pour la pullulanase, l'insertion de ce fragment d'ADN dans un vecteur approprié, l'introduction de ce vecteur dans un hôte approprié ou l'introduction de ce fragment d'ADN dans le chromosome d'un hôte approprié, la culture de cet hôte, l'expression de la pullulanase et la récolte de la pullulanase.

18. Utilisation d'une pullulanase selon l'une quelconque des revendications 1 à 15 pour la saccharification de l'amidon.

19. Molécule d'ADN comprenant la séquence de nucléotides (SEQ ID NO:10) qui code pour la pullulanase selon l'une quelconque des revendications 1 à 15.

20. Molécule d'ADN selon la revendication 19, caractérisée en ce qu'elle comprend tout le gène (SEQ ID NO:8) de la pullulanase de Bacillus deramificans LMG P-13056.

21. Vecteur d'expression contenant la molécule d'ADN selon la revendication 19.

22. Vecteur d'intégration chromosomique contenant la molécule d'ADN selon la revendication 19.

23. Vecteur d'expression caractérisé par la carte de restriction représentée à la figure 1.

24. Vecteur d'intégration chromosomique caractérisé par la carte de restriction représentée à la figure 3.

25. Souche transformée de Bacillus liceniformis comprenant la molécule d'ADN selon la revendication 19.

26. Souche transformée de Bacillus licheniformis comprenant le vecteur d'expression selon la revendication 21 ou le vecteur d'intégration chromosomique selon la revendication 22.

27. Souche transformée de Bacillus licheniformis comprenant le vecteur d'expression caractérisé par la carte de restriction représentée à la figure 1 ou le vecteur d'intégration chromosomique caractérisé par la carte de restriction représentée à la figure 3.

28. Une culture de Bacillus deramificans LMG P-13056.

29. Une culture de Bacillus deramificans LMG P-13056 et culture dérivée ou mutée de celle-ci, susceptible de produire une pullulanase selon l'une quelconque des revendications 1 à 13.

30. Composition enzymatique comprenant une pullulanase selon l'une des revendications 1 à 15.

## Patentansprüche

1. Pullulanase, dadurch gekennzeichnet, daß ihre N-terminale Sequenz (SEQ ID Nr:1) die folgende in Richtung der Amino-Carboxyl-Gruppe und von links nach rechts ist:

Asp Gly Asn Thr Thr Thr Ile Ile Val His
1               5                   10
Tyr Phe Cys Pro Ala Gly Asp Tyr Gln Pro
                15                  20

2. Pullulanase nach Anspruch 1, dadurch gekennzeichnet, daß sie durch den Stamm Bacillus deramificans erzeugt ist.

3. Pullulanase, dadurch gekennzeichnet, daß sie die Aminosäuresequenz von 1 bis 928 Aminosäuren enthält, wie sie in der Fig. 4 veranschaulicht ist (SEQ ID Nr:11).

4. Pullulanase nach Anspruch 3, dadurch gekennzeichnet, daß sie in Form eines Vorläufers synthetisiert ist, welcher eine zusätzliche Sequenz von 29 Aminosäuren enthält (SEQ ID Nr:12).

5. Pullulanase nach Anspruch 1, dadurch gekennzeichnet, daß sie eine optimale enzymatische Aktivität, gemessen bei einer Temperatur von etwa 60°C bei einem pH-Wert von etwa 4,3, entwickelt.

6. Pullulanase nach einem der vorhergehenden Ansprüche, dadurch gekennzeichnet, daß sie ein Molekulargewicht von etwa 100 kDa aufweist.

7. Pullulanase nach einem der vorhergehenden Ansprüche, dadurch gekennzeichnet, daß sie einen isoelektrischen Punkt aufweist, welcher zwischen 4,1 und 4,5 enthalten ist.

8. Pullulanase nach einem der vorhergehenden Ansprüche, dadurch gekennzeichnet, daß sie eine enzymatische Aktivität von mehr als 80% der maximalen enzymatischen Aktivität in einem pH-Bereich, welcher zwischen etwa 3,8 und etwa 4,9 enthalten ist, für eine Temperatur von etwa 60°C entwickelt, wobei die maximale enzymatische Aktivität bei einer Temperatur von 60°C und einem pH-Wert von 4,3 gemessen wird.

9. Pullulanase nach einem der vorhergehenden Ansprüche, dadurch gekennzeichnet, daß sie eine relative enzymatische Aktivität von über 90%, gemessen bei einer Temperatur von etwa 60°C in Abwesenheit von Substrat in einem pH-Bereich, welcher zwischen etwa 3,8 und etwa 5 enthalten ist, zeigt.

10. Pullulanase nach einem der vorhergehenden Ansprüche, dadurch gekennzeichnet, daß sie die glucosidischen α-1,6-Bindungen in Amylopektin hydrolysiert.

11. Pullulanase nach einem der vorhergehenden Ansprüche, dadurch gekennzeichnet, daß sie Pullulan zu Maltotriose abbaut.

12. Pullulanase nach einem der vorhergehenden Ansprüche, dadurch gekennzeichnet, daß sie Amylopektin unter Bildung von Oligosacchariden hydrolysiert.

13. Pullulanase nach einem der vorhergehenden Ansprüche, dadurch gekennzeichnet, daß sie die Kondensationsreaktion von Maltose unter Bildung von Tetrahalosiden katalysiert.

14. Pullulanase nach einem der vorhergehenden Ansprüche, dadurch gekennzeichnet, daß sie ausgehend von dem Stamm Bacillus deramificans LMG P-13056 oder einem Derivat oder Mutanten dieses Stammes erhalten werden kann.

15. Pullulanase nach einem der Ansprüche 1 bis 13, dadurch gekennzeichnet, daß sie auf heterologe Weise durch einen Mikroorganismus der Gattung Bacillus erzeugt wird, welcher ein für eine alkalische Protease codierendes Gen enthält, wenn er sich im Wildzustand befindet, wobei das für die alkalische Protease codierende Gen vom Mikroorganismus der Gattung Bacillus deletiert wurde.

16. Verfahren zur Erzeugung von Pullulanase nach einem der Ansprüche 1 bis 14, dadurch gekennzeichnet, daß es die Kultivierung des Stammes Bacillus deramificans oder eines Derivates dieses Stammes, welche die Pullulanase in einem geeigneten Nährmedium erzeugen können, das Kohlenstoff- und Stickstoffquellen und Mineralsalze enthält, unter aerobiotischen Bedingungen, und die Gewinnung der so erhaltenen Pullulanase umfaßt.

17. Verfahren zur Herstellung einer Pullulanase nach einem der Ansprüche 1 bis 14, dadurch gekennzeichnet, daß es die Isolierung eines DNA-Fragmentes, das für Pullulanase codiert, die Insertion dieses DNA-Fragmentes in einen geeigneten Vektor, das Einführen dieses Vektors in einen geeigneten Wirt oder das Einführen dieses DNA-Fragmentes in das Chromosom eines geeigneten Wirtes, das Züchten dieses Wirtes, die Expression der Pullulanase und die Gewinnung der Pullulanase umfaßt.

18. Verwendung einer Pullulanase nach einem der Ansprüche 1 bis 15 für die Verzuckerung von Amidon.

19. DNA-Molekül, umfassend die Nucleotidsequenz (SEQ ID Nr:10), welche für Pullulanase nach einem der Ansprüche 1 bis 15 codiert.

20. DNA-Molekül nach Anspruch 19, dadurch gekennzeichnet, daß es das gesamte Gen (SEQ ID Nr:8) der Pullulanase von Bacillus deramificans LMG P-13056 umfaßt.

21. Expressionsvektor, enthaltend das DNA-Molekül nach Anspruch 19.

22. Chromosomaler Integrationsvektor, enthaltend das DNA-Molekül nach Anspruch 19.

23. Expressionsvektor, gekennzeichnet durch die in Fig. 1 dargestellte Restriktionskarte.

24. Chromosomaler Integrationsvektor, gekennzeichnet durch die in Fig. 3 dargestellte Restriktionskarte.

25. Transformierter Stamm von Bacillus licheniformis, umfassend das DNA-Molekül nach Anspruch 19.

26. Transformierter Stamm von Bacillus licheniformis, umfassend den Expressionsvektor nach Anspruch 21 oder den chromosomalen Integrationsvektor nach Anspruch 22.

27. Transformierter Stamm von Bacillus licheniformis, umfassend den Expressionsvektor, der durch die in Fig. 1 veranschaulichte Restriktionskarte charakterisiert ist, oder den chromosomalen Integrationsvektor, der durch die in der Fig. 3 veranschaulichte Restriktionskarte gekennzeichnet ist.

28. Eine Kultur von Bacillus deramificans LMG P-13056.

29. Eine Kultur von Bacillus deramificans LMG P-13056 und eine davon abgeleitete oder mutierte Kultur, welche eine Pullulanase nach einem der Ansprüche 1 bis 13 erzeugen kann.

30. Enzymatische Zusammensetzung, umfassend eine Pullulanase nach einem der Ansprüche 1 bis 15.

## Claims

1. Pullulanase, characterised in that it has the following N-terminal sequence (SEQ ID No. 1) in the aminocarboxyl direction, from left to right:

Asp Gly Asn Thr Thr Thr Ile Ile Val His
1               5                   10

Tyr Phe Cys Pro Ala Gly Asp Tyr Gln Pro
                15                  20

2. Pullulanase according to claim 1, characterised in that it is produced by the strain Bacillus deramificans.

3. Pullulanase, characterised in that it comprises the amino acid sequence of 1 to 928 amino acids which is illustrated in Figure 4 (SEQ ID No. 11).

4. Pullulanase according to claim 3, characterised in that it is synthesised in the form of a precursor containing an additional sequence of 29 amino acids (SEQ ID No. 12).

5. Pullulanase according to claim 1, characterised in that it develops optimum enzyme activity, measured at a temperature of approximately 60 °C, at a pH of approximately 4.3.

6. Pullulanase according to any one of the preceding claims, characterised in that it has a molecular weight of approximately 100 kDa.

7. Pullulanase according to any one of the preceding claims, characterised in that it has an isoelectric point of between 4.1 and 4.5.

8. Pullulanase according to any one of the preceding claims, characterised in that it develops enzyme activity of more than 80 % of the maximum enzyme activity within a pH range of between approximately 3.8 and approximately 4.9 in respect of a temperature of approximately 60 °C, the maximum enzyme activity being measured at a temperature of 60 °C and at a pH of 4.3.

9. Pullulanase according to any one of the preceding claims, characterised in that it exhibits relative enzyme activity greater than 90 %, measured at a temperature of approximately 60 °C in the absence of a substrate, within a pH range of between approximately 3.8 and approximately 5.

10. Pullulanase according to any one of the preceding claims, characterised in that it hydrolyses the α-1,6 glucoside bonds present in amylopectin.

11. Pullulanase according to any one of the preceding claims, characterised in that it degrades pullulan into maltotriose.

12. Pullulanase according to any one of the preceding claims, characterised in that it hydrolyses amylopectin to form oligosaccharides.

13. Pullulanase according to any one of the preceding claims, characterised in that it catalyses the maltose condensation reaction to form tetraholosides.

14. Pullulanase according to any one of the preceding claims, characterised in that it can be obtained from the Bacillus deramificans strain LMG P-13056 or a derivative or mutant of said strain.

15. Pullulanase according to any one of claims 1 to 13, characterised in that it is produced heterologously by a microorganism of the genus Bacillus which contains a gene coding for an alkaline protease when it is in the wild state, the gene coding for the alkaline protease having been deleted from the microorganism of the genus Bacillus.

16. Method for producing a pullulanase according to any one of claims 1 to 14 characterised in that it comprises culturing the strain Bacillus deramificans or a derivative of said strain that is capable of producing the pullulanase in a suitable nutrient medium containing sources of carbon and nitrogen and minerals salts under aerobiotic conditions, and harvesting the pullulanase thereby obtained.

17. Method for the preparation of a pullulanase according to any one of claims 1 to 14, characterised in that it comprises isolating a DNA fragment coding for the pullulanase, inserting said DNA fragment into a suitable vector, introducing said vector into a suitable host or introducing said DNA fragment into the chromosome of a suitable host, culturing said host, expressing the pullulanase and harvesting the pullulanase.

18. Use of a pullulanase according to any one of claims 1 to 15 for the saccharification of starch.

19. DNA molecule comprising the nucleotide sequence (SEQ ID No. 10) that codes for the pullulanase according to any one of claims 1 to 15.

20. DNA molecule according to claim 19, characterised in that it comprises the whole gene (SEQ ID No. 8) of the pullulanase of Bacillus deramificans LMG P-13056.

21. Expression vector containing the DNA molecule according to claim 19.

22. Chromosomal integration vector containing the DNA molecule according to claim 19.

23. Expression vector characterised by the restriction map represented in Figure 1.

24. Chromosomal integration vector characterised by the restriction map represented in Figure 3.

25. Transformed strain of Bacillus licheniformis comprising the DNA molecule according to claim 19.

26. Transformed strain of Bacillus licheniformis comprising the expression vector according to claim 21 or the chromosomal integration vector according to claim 22.

27. Transformed strain of Bacillus licheniformis comprising the expression vector characterised by the restriction map represented in Figure 1 or the chromosomal integration vector characterised by the restriction map represented in Figure 3.

28. A culture of Bacillus deramificans LMG P-13056.

29. A culture of Bacillus deramificans LMG P-13056 and culture derived or mutated therefrom, capable of producing a pullulanase according to any one of claims 1 to 13.

30. Enzyme composition comprising a pullulanase according to one of claims 1 to 15.
